# EUROPEAN PATENT SPECIFICATION

(11) **EP 2 582 811 B1**
(45) Date of publication and mention of the grant of the patent: **18.09.2019**
(21) Application number: 11725457.3
(22) Date of filing: 15.06.2011
(51) Int. Cl.: C12P 21/02, C12P 21/00, C12N 9/12, C12N 9/16, C12N 15/82, C12N 15/81

(54) **INCREASED PROTEIN EXPRESSION THROUGH INCREASED MEMBRANE FORMATION**
VERSTÄRKTE REKOMBINANTE PROTEINEXPRESSION DURCH MEMBRANBILDUNG
EXPRESSION PROTEINIQUE ACCRUE A CONSEQUENCE D'UNE FORMATION DE MEMBRANE ACCRUE

(30) Priority: 17.06.2010 EP 10166271
(43) Date of publication of application: 24.04.2013
(73) Proprietor: VIB vzw, 9052 Ghent (BE); Universiteit Gent, 9000 Gent (BE)
(72) Inventor: CALLEWAERT, Nico, B-9850 Nevele (BE); GUERFAL, Mouna, B-9031 Drongen (BE)
(86) International application number: PCT/EP2011/059959
(87) International publication number: WO 2011/157761

(56) References cited:
- CARMAN G M ET AL: "Roles of phosphatidate phosphatase enzymes in lipid metabolism", TRENDS IN BIOCHEMICAL SCIENCES, ELSEVIER, HAYWARDS, GB, vol. 31, no. 12, 1 December 2006 (2006-12-01), pages 694-699, XP025132570, ISSN: 0968-0004, DOI: 10.1016/J.TIBS.2006.10.003 [retrieved on 2006-12-01]
- SINIOSSOGLOU SYMEON: "Lipins, lipids and nuclear envelope structure.", TRAFFIC (COPENHAGEN, DENMARK) SEP 2009 LNKD- PUBMED:19490535, vol. 10, no. 9, September 2009 (2009-09), pages 1181-1187, XP002662883, ISSN: 1600-0854
- HAN G-S ET AL: "An unconventional diacylglycerol kinase that regulates phospholipid synthesis and nuclear membrane growth", JOURNAL OF BIOLOGICAL CHEMISTRY 20080718 AMERICAN SOCIETY FOR BIOCHEMISTRY AND MOLECULAR BIOLOGY INC. US, vol. 283, no. 29, 18 July 2008 (2008-07-18), pages 20433-20442, XP002662884, DOI: DOI:10.1074/JBC.M802903200
- MADZAK C ET AL: "Heterologous Protein Expression and Secretion in the Non-conventional Yeast Yarrowia lipolytica: A Review", JOURNAL OF BIOTECHNOLOGY, ELSEVIER SCIENCE PUBLISHERS, AMSTERDAM, NL, vol. 109, no. 1-2, 1 April 2004 (2004-04-01), pages 63-81, XP002995176, ISSN: 0168-1656, DOI: 10.1016/J.JBIOTEC.2003.10.027
- Laura O ' Hara ET AL: "Control of Phospholipid Synthesis by Phosphorylation of the Yeast Lipin Pah1p/Smp2p Mg 2+ -dependent Phosphatidate Phosphatase *", EMBO J. J. Biol. Chem, 10 November 2006 (2006-11-10), pages 9210-9218, XP055165200, Retrieved from the Internet: URL:http://www.ncbi.nlm.nih.gov/pmc/articl es/PMC1769310/pdf/nihms14058.pdf [retrieved on 2015-01-27]
- Helena Santos-Rosa ET AL: "The yeast lipin Smp2 couples phospholipid biosynthesis to nuclear membrane growth , Sew Peak-Chew", , 1 January 2005 (2005-01-01), XP055165204, Retrieved from the Internet: URL:http://www.ncbi.nlm.nih.gov/pmc/articl es/PMC1142606/pdf/7600672a.pdf [retrieved on 2015-01-27]
- P. FAGONE ET AL: "Phospholipid Biosynthesis Program Underlying Membrane Expansion during B-lymphocyte Differentiation", JOURNAL OF BIOLOGICAL CHEMISTRY, vol. 282, no. 10, 25 August 2006 (2006-08-25), pages 7591-7605, XP055284961, US ISSN: 0021-9258, DOI: 10.1074/jbc.M608175200

## Description

### Field of the invention

The present application relates to the field of protein expression technologies. More specifically, it is demonstrated that cells with increased levels of phosphatidic acid, which can be achieved through either or both of phosphatidic acid inhibition and diacylglycerol kinase overexpression, display increased membrane formation from which increased levels of proteins can be recovered.

### Background

The last years an increasing interest has grown to isolate and characterize membrane proteins. Consortia have been created which study is entirely focused on the expression of membrane proteins and this in different expression systems. One example is the MEPNET (Membrane Protein Network) consortium. The high interest in membrane proteins is due to the fact that they play an important role in several biological processes such as ion transport, recognition of molecules, signal transduction etc. GPCRs (G-protein coupled receptors) are the major class of pharmacological interesting membrane proteins. These proteins with 7 transmembrane regions are involved in recognition of signals as diverse as light (rhodopsin), smells (olfactory receptors), neurotransmitters and hormones. The human genome, for instance, encodes hundreds of these receptors and in view of their crucial role in the 'sensing' of numerous signals and because of their accessible localization on the surface of the membrane, these proteins are the target of a variety of pharmacological compounds. More than 40% of the drugs on the market are directed to specific GPCRs.

Although membrane proteins represent 20-30% of all genes in prokaryotes as well as in eukaryotes, only little is known about structure and function relationship of membrane proteins. One reason is their low abundance in native tissue leading to the fact that not enough material can be isolated for structural studies. By way of example, only for six GPCRs the structure has been characterized, two of which in the last year: rhodopsin, the β1 and β2 adrenergic receptors, the adenosine 2A receptor, the CXCR4 receptor and the dopamine D3 receptor. It is no coincidence that exactly these 6 belong to the small group of easily obtainable GPCRs. Rhodopsin was purified from cow eyes, where it naturally occurs in high concentrations - a lone exception to the rule of low expression levels - and as a result it was the first GPCR for which a structure was determined (Palczewski et al., 2000; Salom et al., 2006). The β1 and β2 adrenergic receptors and the adenosine 2A receptors were known to those of skill in the art to be intrinsically fairly easily expressible (Lundstrom et al., 2006) and their structures were published in 2007-2008 (Cherezov et al., 2007; Rasmussen et al., 2007; Jaakola et al., 2008; Warne et al., 2008). The two most recent crystal structures are those of the CXCR4 receptor (Wu et al., 2010) and the dopamine D3 receptor (Chien et al., 2010). Most GPCRs however, express at levels that are 10 to 1000-fold lower than those of the crystallized receptors in non-engineered eukaryotic cells. Consequently, fundamental biotechnological work is necessary to increase the expression levels for the majority of these receptors to those required for biophysical characterization.

Indeed, techniques used to obtain high resolution 3D structures such as NMR and X-ray crystallography, require large amounts (milligrams) of purified proteins. A challenge therefore is to produce these proteins to levels high enough to allow structural studies. GPCRs have been successfully overexpressed in bacteria, yeast, mammalian cell lines and insect cells. Expression levels although are still rather low and for most proteins a 5 to 10-fold increase in expression level would lead to sufficient material to allow isolation of the protein for structural studies.

Expression of eukaryotic membrane proteins in prokaryotic systems mostly leads to poor expression levels and in many cases the protein ends up in denatured form in inclusion bodies. Expression of the rat neurotensin receptor (Grisshammer et al., 1993) and the human adenosine A2A receptor are examples of the few successfully overexpressed GPCRs in *E.coli.* Expression in yeast is a valuable alternative for the expression of membrane proteins. Yeast cells are easy to handle, and can grow in fermentors to very high cell densities. Different techniques to increase the expression levels of the membrane protein have been used in yeast such as lowering the induction temperature, adding antagonist DMSO or histidine to the induction medium (André et al., 2006). None of these techniques however markedly increased the total yield of the expressed protein but improved significantly the functionality of the protein. This is reflected by the fact that only very few of these membrane proteins have been crystallized, even though 30% of eukaryotic genes code for these proteins. Only three yeast species are currently used for the production of membrane proteins, namely *Saccharomyces cerevisiae, Schizosaccharomyces pombe* and *Pichia pastoris. P. pastoris* is the most successfully used yeast which is reflected in the amount of membrane protein structures present in the membrane protein data base.

Another yeast of biotechnological importance is *Yarrowia lipolytica.* It has good capacities for the secretion of heterologous proteins as shown by the number of proteins expressed in this yeast (Madzak et al., 2004). *Yarrowia lipolytica* is an oleaginous yeast isolated predominantly from lipid- or protein containing sources such as cheese, yoghurts, kefir and meat. It is a dimorphic fungus capable to grow as a yeast cell or hyphae or pseudohyphae. A wide range of biotechnological tools for yeast manipulation are available (Madzak et al., 2004). This unconventional yeast has however not yet been exploited for the production of membrane proteins.

Thus, it would be advantageous to have expression systems that permit higher expression of proteins, particularly of membrane proteins, so that sufficient quantities can be made allowing biophysical characterization and crystallization of these proteins.

### Summary

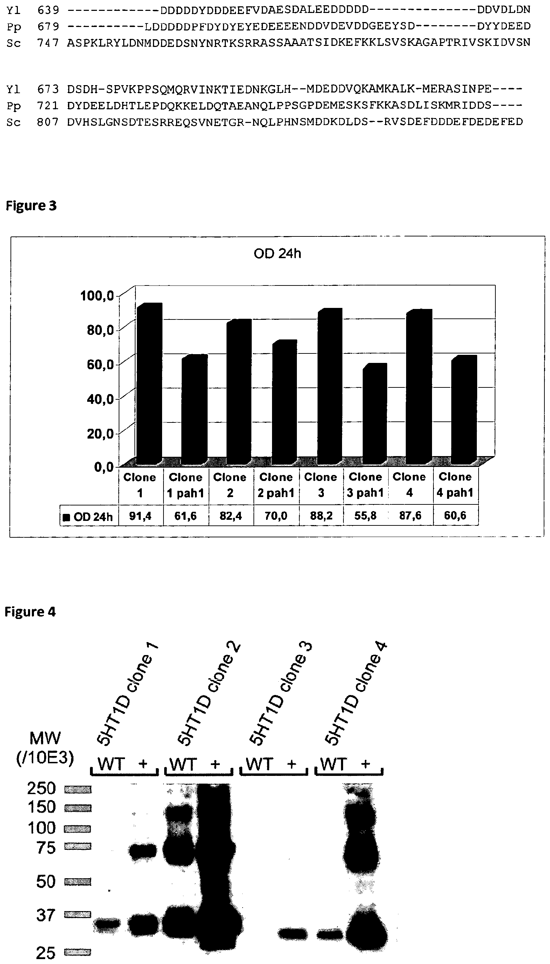

The present specification describes cells allowing higher expression of proteins, particularly membrane proteins, and methods of expressing proteins in these cells. This is achieved by derepression of phospholipid synthesis, particularly derepression of nuclear and/or endoplasmic reticulum (ER) phospholipid synthesis. The derepression of phospholipid synthesis genes can be achieved by elevating phosphatidic acid (PA) content at the nuclear/ER membrane. This, in turn, is achieved by inhibiting phosphatidic acid phosphatase activity and/or upregulation of diacylglycerol kinase activity, which results in expansion of the nuclear/ER membrane. Surprising in view of the significantly changed phospholipid composition as a result of increased PA levels (Han et al., 2006; Han et al., 2007) is that these membrane expansions can accommodate huge amounts of proteins, while the cells remain viable. Indeed, it is shown herein that derepression of ER phospholipid synthesis allows a 10-fold and higher increase in production of e.g. GPCRs (e.g. Figure 4).

Accordingly, the present invention provides subject-matter as set forth in any one and all of (1) to (21) below:
(1) A method of producing a protein in a eukaryotic cell, comprising the steps of: providing a eukaryotic cell wherein:
   - expression and/or activity of an endogenous phosphatidic acid phosphatase is inhibited; and/or
   - overexpressing a diacylglycerol kinase
   to the extent that the endoplasmic reticulum is expanded in the cell, the cell further comprising an endogenous nucleic acid sequence under control of an exogenous promoter, said nucleic acid encoding a protein, or the cell further comprising an exogenous nucleic acid sequence encoding a receptor or secreted protein, in conditions suitable for expressing the protein.
(2) The method of (1), wherein the endogenous phosphatidic acid phosphatase is PAH1 or a homolog thereof.
(3) The method of (1) or (2), wherein the expression and/or activity of the endogenous phosphatidic acid phosphatase is inhibited through disruption of the endogenous phosphatidic acid phosphatase gene at nucleic acid level.
(4) The method of (1) or (2), wherein the expression and/or activity of the endogenous phosphatidic acid phosphatase is inhibited through an inhibitory RNA directed to the endogenous phosphatidic acid phosphatase gene transcript.
(5) The method of any one of (1)-(4), wherein the diacylglycerol kinase is DGK1 or a homolog thereof.
(6) The method of any one of (1)-(5), wherein endogenous diacylglycerol kinase is overexpressed.
(7) The method of any one of (1)-(5), wherein an exogenous diacylglycerol kinase is overexpressed.
(8) The method of any one of (1)-(7), wherein the overexpression is inducible.
(9) The method of any one of (1)-(8), wherein the protein is a membrane-bound protein.
(10) The method of any one of (1)-(9), wherein the protein is a receptor, in particular a GPCR.
(11) The method of any one of (1)-(10), wherein the eukaryotic cell is a yeast cell, in particular a Yarrowia or Pichia cell, and the protein is to be expressed and isolated from the cell.
(12) The method of (11), wherein the yeast cell is a glyco-engineered yeast cell.
(13) The method of any one of (1)-(10), wherein the eukaryotic cell is a mammalian cell, in particular a Hek293S cell.
(14) The method of any one of (1)-(13), further comprising the step of isolating the expressed protein.
(15)A method of producing a virus-like particle in a eukaryotic cell, comprising the steps of: providing a eukaryotic cell wherein:
   - expression and/or activity of an endogenous phosphatidic acid phosphatase is inhibited; and/or
   - overexpressing a diacylglycerol kinase,
   to the extent that the endoplasmic reticulum is expanded in the cell, the cell comprising one or more nucleic acid sequences encoding the one or more proteins making up the virus-like particle, in conditions suitable for expressing the one or more proteins.
(16) The method of (15), wherein the virus-like particle further encompasses lipids, such as in a lipoparticle.
(17) The method of (15) or (16), wherein the virus-like particles are used for production of vaccines or for production of membrane proteins.
(18) The method of any one of (15)-(17), wherein the virus-like particles are based on a hepatitis virus, particularly HCV virus, on an encephalitis virus, particularly Japanese encephalitis virus, or on a Dengue virus.
(19) A eukaryotic cell deficient in expression and/or activity of an endogenous phosphatidic acid phosphatase, and/or overexpressing an endogenous diacylglycerol kinase by means of an exogenous promoter and/or genetically engineered so as to express a diacylglycerol kinase that said cell does not normally express, to the extent that the endoplasmic reticulum is expanded in the cell, the cell further comprising an endogenous nucleic acid sequence under control of an exogenous promoter or further comprising an exogenous nucleic acid sequence encoding a receptor or secreted protein to be expressed and isolated from the cell.
(20) The eukaryotic cell of (19), wherein the cell is a yeast cell, in particular a Yarrowia or Pichia cell.
(21) The eukaryotic cell of (19) or (20), wherein the endogenous phosphatidic acid phosphatase is PAH1 or a homolog thereof and/or wherein the diacylglycerol kinase is DGK1 or a homolog thereof.
(22) A cell culture of cells of any one of (19)-(21).

The specification describes methods of enhancing production of proteins in a eukaryotic cell, which entail that a eukaryotic cell deficient in expression and/or activity of an endogenous phosphatidic acid phosphatase, and/or overexpressing a diacylglycerol kinase is provided, wherein the cell comprises a nucleic acid sequence encoding the protein of interest and the cell is maintained in conditions suitable for expressing the protein. Afterwards, the protein or proteins of interest can then be recovered from the cell. Typically, the nucleic acid sequence encoding the protein to be produced is an exogenous nucleic acid sequence or an endogenous nucleic acid sequence under control of an exogenous promoter. The protein may be expressed constitutively or in an inducible way. Accordingly, the promoter may be a constitutive or inducible promoter.

The present specification also describes that the endogenous phosphatidic acid phosphatase may be PAH1 or a homolog thereof. The cell may be deficient in expression and/or activity of the endogenous phosphatidic acid phosphatase through disruption of the endogenous phosphatidic acid phosphatase gene at nucleic acid level. Alternatively, the cell may be deficient in expression and/or activity through an inhibitory RNA directed to the endogenous phosphatidic acid phosphatase gene transcript. The deficiency of the expression and/or activity of the endogenous phosphatidic acid phosphatase may be inducible, which is envisaged in current application.

The present specification further describes that the diacylglycerol kinase that is overexpressed may be DGK1 or a homolog thereof. The diacylglycerol kinase that is overexpressed may be an endogenous diacylglycerol kinase or an exogenous diacylglycerol kinase. Typically, although not necessarily, the promoter driving the diacylglycerol kinase expression may be an exogenous promoter. Overexpression of the diacylglycerol kinase may be constitutive or inducible. Likewise, the promoters driving the diacylglycerol kinase expression may be constitutive or inducible promoters.

The present specification further describes, that the proteins that are produced in the eukaryotic cells described herein may be membrane-bound proteins. Said protein may be a receptor or more particular a GPCR. Importantly, however, the methods may also be used to improve production of non-membrane-bound proteins, as the ER is also the place where secreted proteins are synthesized. The present specification also describes that more than one, i.e. two or more different proteins may be produced simultaneously.

The present specification further describes that the eukaryotic cells used for protein production may be yeast cells. The yeast cells may be methylotrophic yeast cells, such as species of the genus *Hansenula* (e.g. *Hansenula polymorpha*), species of the genus *Candida* (e.g. *Candida boidinii)* or most particularly species of the genus *Pichia,* such as *Pichia pastoris.* The yeast cells may be of the genus *Yarrowia,* most particularly of the species *Yarrowia lipolytica.* The present specification also describes that the yeast cells may not be from the species *Saccharomyces cerevisiae* or even not from the genus *Saccharomyces.* The present specification further describes, that the yeast cells may be glyco-engineered yeast cells. Examples thereof include, but are not limited to, yeast cells that have humanized glycosylation patterns. Typically, the protein that may be expressed in a yeast cell will be isolated (or possibly secreted) from the cell.
The present specification further describes, that the eukaryotic cells may be plant cells, particularly plant cell cultures. The eukaryotic cells may be also mammalian cells, most particularly Hek293 cells, such as Hek293S cells (Reeves et al., 1996; Reeves et al., 2002).

The present specification further describes that the methods of protein production may comprise the step of isolating the expressed protein. This typically involves recovery of the material wherein the protein is present (e.g. a cell lysate or specific fraction thereof, the medium wherein the protein is secreted) and subsequent purification of the protein. Means that may be employed to this end are known to the skilled person and include specific antibodies, tags fused to the proteins, affinity purification columns, and the like.

The present specification also describes that the methods can be used to produce virus-like particles or VLPs. Indeed, the expression of viral structural proteins, such as Envelope or Capsid, can result in the self-assembly of VLPs. Thus, the cells may comprise one or more nucleic acid sequences encoding the one or more proteins making up the virus-like particle, in conditions suitable for expressing the one or more proteins. The present specification also describes that where the VLP consists of more than one protein, the different proteins can also be expressed as a single polyprotein. This was demonstrated previously for different viruses (Bräutigam et al., 1993; Kibenge et al., 1999).

Remarkable about VLPs is their ease of production: not only can they be produced in a variety of cell culture systems including mammalian cell lines, insect cell lines, yeast, and plant cells as well as whole plants (Santi et al., 2006), but the individual viruses they are derived from are also extremely diverse in terms of structure and include viruses that have a single capsid protein, multiple capsid proteins, and those with and without lipid envelopes (Noad and Roy, 2003). VLPs typically bud from cellular membranes (and are typically also secreted by the cell). The virus-like particle may encompass lipids in addition to protein(s). This is the case for VLPs derived from viruses with lipid envelopes, but also for lipoparticles (which are engineered to express membrane proteins, see e.g. Endres et al., 1997; Balliet and Bates, 1998; Willis et al., 2008; and commercially available from Integral Molecular, Philadelphia). VLPs can thus efficiently be used for production of (membrane) proteins, but also in the development of vaccines (Noad and Roy, 2003; Roy and Noad, 2008). Particular VLPs that are envisaged for vaccine production include, but are not limited to, virus-like particles based on a hepatitis virus, particularly HBV or HCV virus, on a HIV virus, on an encephalitis virus, particularly Japanese encephalitis virus, or on a Dengue virus.

The present specification further describes, eukaryotic cells that are deficient in expression and/or activity of an endogenous phosphatidic acid phosphatase, and/or that overexpress a diacylglycerol kinase, wherein if the eukaryotic cell is a *Saccharomyces* cell, it comprises an exogenous nucleic acid sequence, or an endogenous nucleic acid sequence under control of an exogenous promoter, encoding a protein to be expressed and isolated from the cell. These cells can be used in the methods of enhancing protein production described herein.

The present specification further describes, that the eukaryotic cells may be yeast cells. The yeast cells may be methylotrophic yeast cells, such as species of the genus *Hansenula* (e.g. *Hansenula polymorpha*), species of the genus *Candida* (e.g. *Candida boidinii)* or most particularly species of the genus *Pichia,* such as *Pichia pastoris.* The yeast cells can also be of the genus *Yarrowia,* most particularly of the species *Yarrowia lipolytica.* The present specification also describes that the yeast cells may be not from the species *Saccharomyces cerevisiae* or even not from the genus *Saccharomyces.* The yeast cells may be glyco-engineered yeast cells. Examples thereof include, but are not limited to, yeast cells that have humanized glycosylation patterns.

As described for the methods above, in the eukaryotic cells the endogenous phosphatidic acid phosphatase may be PAH1 or a homolog thereof and/or the diacylglycerol kinase particularly may be DGK1 or a homolog thereof.

The present specification describes that the eukaryotic cells may further comprise an exogenous nucleic acid sequence, or an endogenous nucleic acid sequence under control of an exogenous promoter, encoding a protein to be expressed and isolated from the cell. This protein may be a membrane-bound protein or a secreted protein. The protein may be a receptor, more particularly a GPCR.

The present specification finally describes a cell culture of the eukaryotic cells as described herein.

### Brief description of the Figures

Figure 1: Sequence of *Yarrowia lipolytica* PAH1. **A**, the nucleotide sequence of the *Yarrowia lipolytica* PAH1 gene with 800 bp surrounding genomic region at either side (SEQ ID NO: 1). Primers used to isolate the promoter and terminator regions of the gene are indicated. **B,** the amino acid sequence of the PAH1 protein of *Yarrowia lipolytica* is shown (SEQ ID NO: 2). The characteristic sequence of the HAD_like domain is indicated in pink, the conserved N-terminal lipin domain in blue.

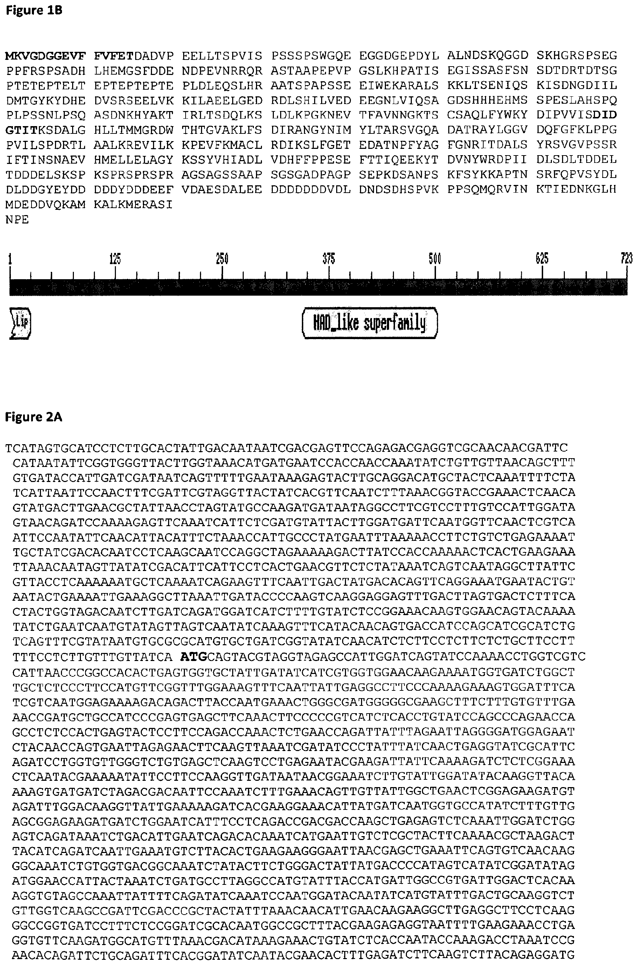

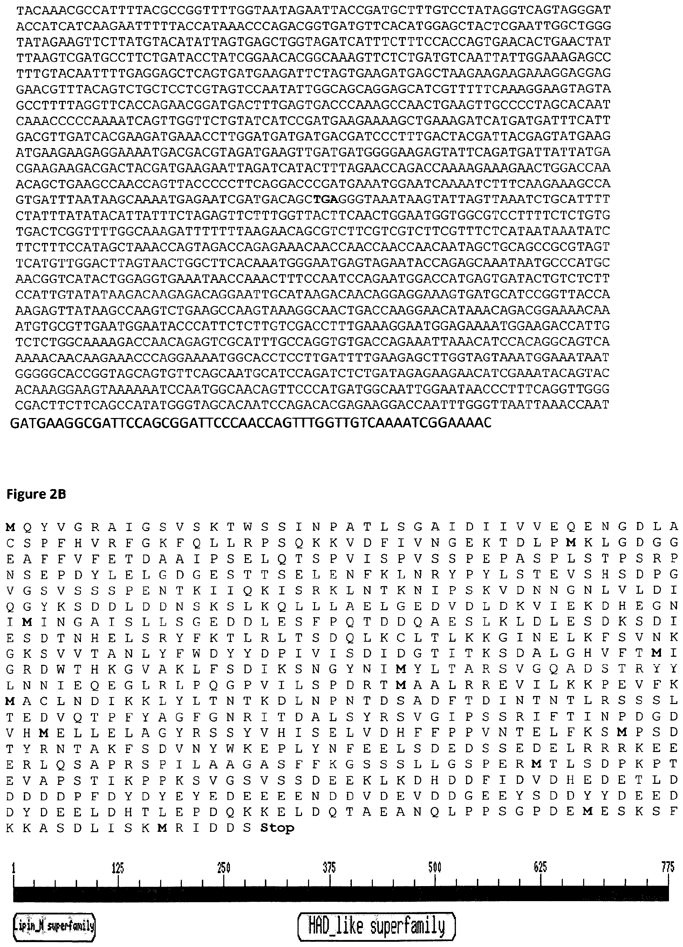

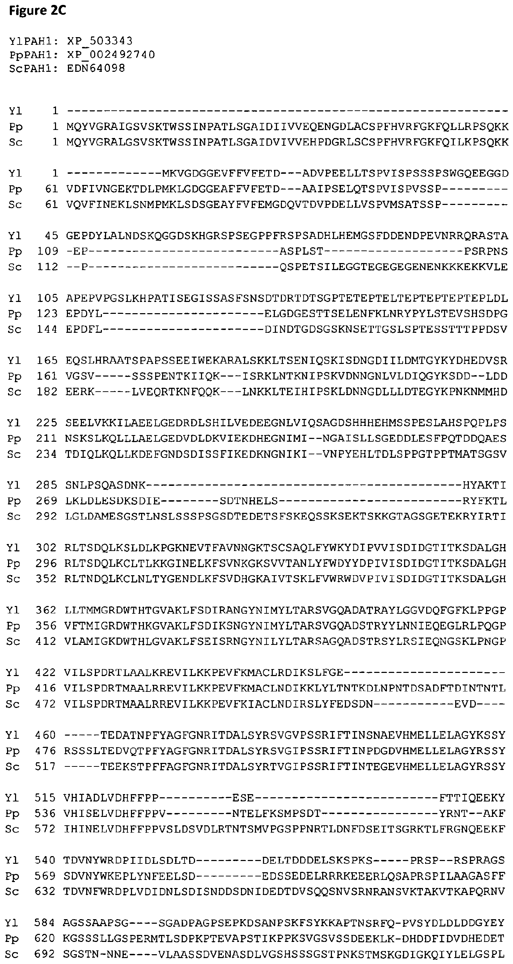

Figure 2: Sequence of *Pichia pastoris* PAH1. **A**, the nucleotide sequence of the *Pichia pastoris* PAH1 gene with 1000 bp surrounding genomic region at either side (SEQ ID NO: 3). Taken from IG-66 strain sequence, contig1302_7261_4937; the start and stop codon are indicated in green and red, respectively. **B,** the amino acid sequence of the PAH1 protein of *Pichia pastoris* is shown (SEQ ID NO: 4). The HAD_like domain is indicated in pink, the conserved N-terminal lipin domain in blue. **C**, protein sequence alignment of the *Saccharomyces cerevisiae, Pichia pastoris,* and *Yarrowia lipolytica* PAH1 protein.
Figure 3: Comparison of the growth of PAH1 knock out strains versus the respective wild type strain. ΔPAH1 strains tend to show a slower growth.
Figure 4: Expression of GPCRs in PAH1 knock out strains. The Western blot shows that the PAH1 knock out strains express higher levels of the 5HT1D receptor compared to the wild type strains. Microsomes were isolated and equal quantities of total membrane protein were loaded. At least 10-fold increase of the 5HT1D receptor can be observed. As usual, dimers and higher oligomers of the receptor are also seen. Clone 1 and 2 are clones bearing 3 copies of the 5HT1D receptor which is expressed starting from the lip2 prepro sequence of *Y. lipolytica.* Clone 3 (1 copy) and 4 (2 copies) express the receptor with its own signal sequence.
Figure 5: TEM picture of *Yarrowia lipolytica* with disruption in the PAH1 gene. The magnification shows the massive proliferation of tightly stacked membranes, continuous with the ER.
Figure 6: sequences of yeast DGK1. **A**, **D**: nucleotide (SEQ ID NO: 5) and amino acid (SEQ ID NO: 6) sequence of *Yarrowia lipolytica* DGK1, respectively. **B, E**: nucleotide (SEQ ID NO: 7) and amino acid (SEQ ID NO: 8) sequence of *Pichia pastoris* DGK1, respectively. **C**, **F**: nucleotide (SEQ ID NO: 9) and amino acid (SEQ ID NO: 10) sequence of *Saccharomyces cerevisiae* DGK1, respectively. **G**, protein sequence alignment of the *Pichia pastoris, Saccharomyces cerevisiae* and *Yarrowia lipolytica* DGK1 protein.
Figure 7: expression of GPCRs in DGK1 overexpression strains. Western blotting of 3 *Pichia pastoris* strains overexpressing the dgk1p together with the A2A receptor. Equal amounts of protein were loaded on the gel. Blot shows a higher expression level for the dgk1 overexpressing strains (clone 1-3) compared to the wild type strain (WT).
Figure 8: Comparison of the growth of a DGK1 overexpressing strain versus the respective wild type strain. Growth rates are comparable.
Figure 9: TEM picture of *Pichia pastoris* strains overexpressing the DGK1 gene. The arrows show the membranes induced by the DGK1p overexpression.
Figure 10 shows the cloning strategy used for disruption of the PAH1 gene of *Pichia pastoris.*
Figure 11: Growth analysis of the *Pichia pastoris* strain with a disrupted PAH1 gene and the corresponding wild type strain. Cells are grown to saturation, diluted to 2.10e⁷ cells/ml and serial dilutions (1:10) of the cells are spotted (5 µl). Top panel: growth at 30°C; bottom panel: growth at 37°C.
Figure 12: TEM picture of *Pichia pastoris* strains with disruption in the PAH1 gene. The arrows show the membranes induced by the lack of PAH1.
Figure 13: Electron microscopy pictures of ΔPAH1 *P. pastoris* cells on glucose and oleic acid.
   ΔPAH1 cells grown on glucose show extra ER membrane which are mostly internalized in vacuoles. The PAH1 KO strain grown on oleic acid has a much more pronounced increase in ER membrane which are not present in vacuoles. A strategy to increase the effect of the PAH1 KO on membrane protein expression might be to express the receptor from a fatty acid inducible promoter.

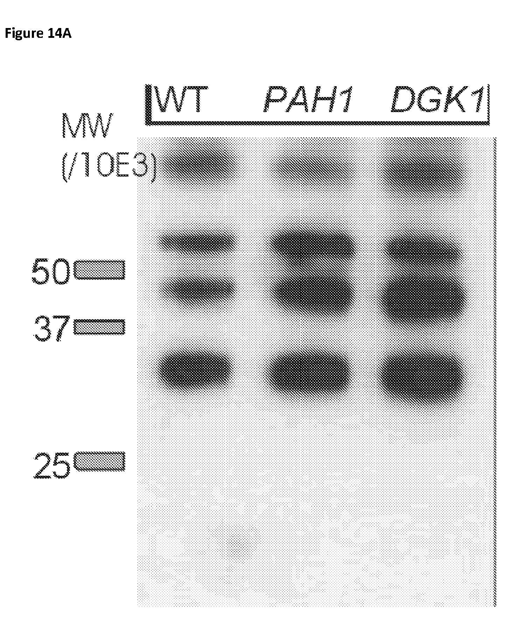

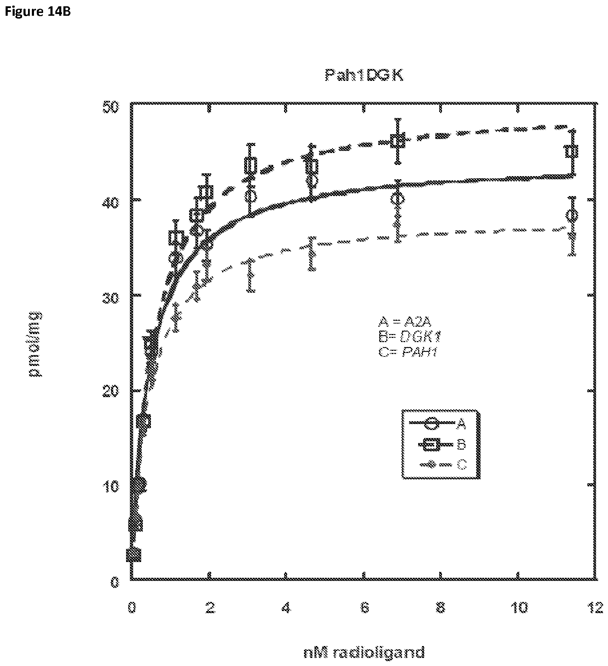

Figure 14: Expression and functionality of GPCRs in PAH1 knock out and DGK1 overexpression strains of *Pichia pastoris.* **A,** Comparison of the expression levels of the A2AR in the WT, PAH1 and DGK1 strain in *Pichia pastoris.* B, Radioligand binding studies on membranes of *P. pastoris* cells expressing the A2AR. 5 µg of total membrane protein was incubated with different concentrations of the A2AR antagonist ³[H]ZM241385 in 500 µl binding buffer. Non-specific binding was determined in the presence of 10 mM theophylline. Bound and free ligand were separated on Whatmann GF/B filters using a Brandel harvester. Data were analyzed with the software KaleidaGraph from Synergy Software.

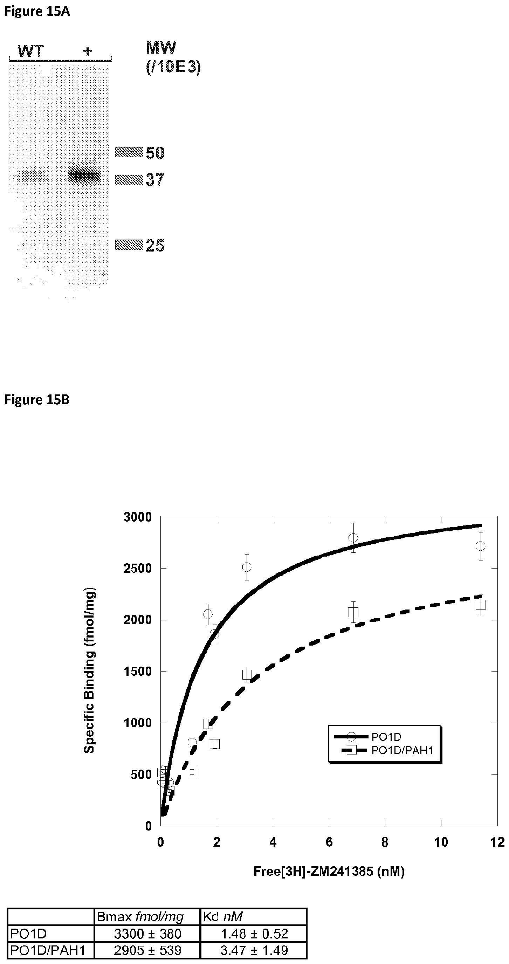

Figure 15: Expression and functionality of GPCRs in a PAH1 knock out strain of *Yarrowia lipolytica.* **A**,Western blot analysis of the Adenosine A2A receptor in Yarrowia lipolytica. Each lane corresponds with 5 µg total membrane protein. Lane 1 shows the expression level of the wild type strain and lane 2 the expression level for the knock out strain. A clear increase in expression level is seen in the PAH1 knock out strain. **B,** saturation binding assay on 5 µg total membrane protein from Y. lipolytica cells expressing the A2A receptor. Conditions are the same as for Figure 14B.

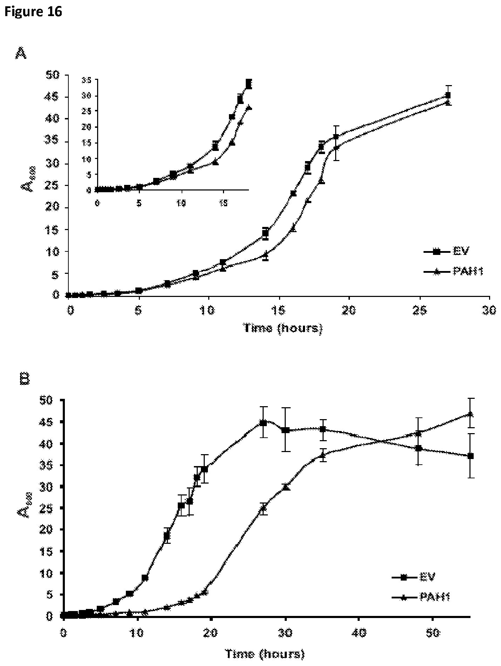

Figure 16: Growth analysis of a *Yarrowia lipolytica* PAH1 knock out strain on glucose and oleic acid. Yeast cells were grown overnight in YPD and than inoculated at an OD 0.1 in YPO (1% yeast extract/ 2% peptone/ 2% oleic acid) or YPD. Cells were incubated at 28°C with continuous shaking at 250 rpm. At the indicated time points samples were taken for absorbance measurements. Data points represent the average of three measurements. A, growth on YPD for 27 hours. B, growth on oleic acid 55 h.

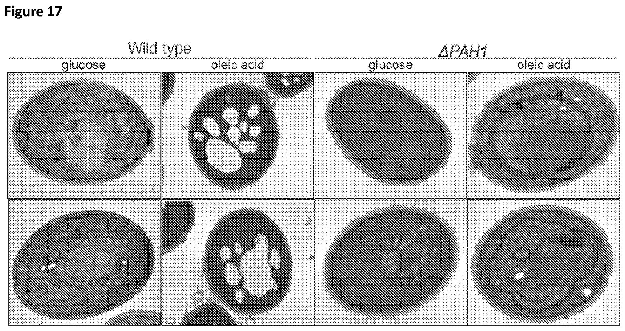

Figure 17: Electron microscopy pictures taken at the end of A2A receptor induction. Yarrowia lipolytica PAH1 knock out strains show a clear increase in ER membrane surface. Especially the strains grown on oleic acid have multiple layers of induced ER membrane. The PAH1 strain grown on oleic acid has no or little lipid droplets compared to the wild type strain.
Figure 18: A. Western blot analysis of A2A receptor overexpressing strains Lane 1 the EV strain not expressing the A2A receptor. Lane 2 represents the strain expressing the A2A receptor from the POX2 promoter. Lane 3 is the PAH1 KO strain. Lanes 4 and 5 show the A2A expression from the hp4d promoter in a wild type and KO strain respectively.

### B. Ligand binding tests on the A2A receptor

Panels on the left show ligand binding tests of the A2A receptor expressed from the oleic acid inducible promoter POX2. Graphs are shown for 2 independent experiments. No binding was obtained for the wild type strain expressing the A2A receptor despite the fact that expression was observed on western blot (Fig 18 A lane 2). Knocking out the PAH1 gene in this strain increases the expression which is reflected in binding of the A2A receptor ligand ZM241385.
Panels on the right show ligand binding results of the A2A receptor expressed from the hp4d promoter. Again graphs show 2 independent experiments. The PAH1 strain shows more receptor binding than the wild type strain.

### Detailed description

### Definitions

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The following terms or definitions are provided solely to aid in the understanding of the invention. Unless specifically defined herein, all terms used herein have the same meaning as they would to one skilled in the art of the present invention. Practitioners are particularly directed to Sambrook et al., Molecular Cloning: A Laboratory Manual, 2nd ed., Cold Spring Harbor Press, Plainsview, New York (1989); and Ausubel et al., Current Protocols in Molecular Biology (Supplement 47), John Wiley & Sons, New York (1999), for definitions and terms of the art. The definitions provided herein should not be construed to have a scope less than understood by a person of ordinary skill in the art.

A "eukaryotic cell" as used herein is a cell containing a nucleus and an endoplasmic reticulum or ER, which is involved in protein transport and maturation.
The term "endogenous" as used herein, refers to substances (e.g. genes) originating from within an organism, tissue, or cell. Analogously, "exogenous" as used herein is any material originated outside of an organism, tissue, or cell, but that is present (and typically can become active) in that organism, tissue, or cell.

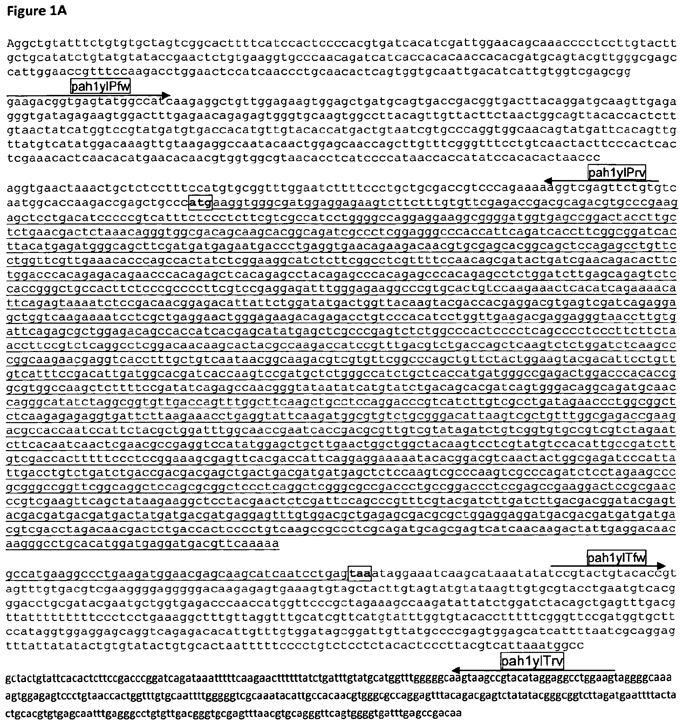

The term "phosphatidic acid phosphatase" or "PAP" is used herein to designate an enzyme catalyzing the dephosphorylation of phosphatidic acid (EC 3.1.3.4), thereby yielding diacylglycerol (DAG) and Pᵢ. Most particularly, PAP is specific for PA and requires Mg²⁺ for activity, to distinguish from lipid phosphate phosphatase, also designated as PAP2, which is not specific for phosphatidic acid and does not require Mg²⁺ for activity (although it helps in reaching maximal activity) (Carman and Han, 2006). The term "PAH1" as used herein refers to the yeast PAP enzyme and the encoding gene (Gene ID: 855201 in *Saccharomyces cerevisiae;* gene and protein sequences of the *Yarrowia lipolytica* and *Pichia pastoris* PAH1 are shown in Figures 1 and 2, including an alignment with the *Saccharomyces cerevisiae* PAH1 protein), sometimes also indicated as SMP2 (Santos-Rosa et al., 2005; Han et al., 2006).
A "PAH1 homolog" as used throughout the application refers to genes and proteins in species other than yeast homologous to PAH1 and having PAP activity. Homology is expressed as percentage sequence identity (for nucleic acids and amino acids) and/or as percentage sequence similarity (for amino acids). Preferably, homologous sequences show at least 50%, at least 60%, at least 70%, at least 80%, at least 90%, at least 95% or at least 99% sequence identity at nucleic acid level or sequence identity or similarity at amino acid level. Algorithms to determine sequence identity or similarity by sequence alignment are known to the person skilled in the art and include for instance the BLAST program. Alternatively, homologs can be identified using the HomoloGene database (NCBI) or other specialized databases such as for instance HOGENOM or HOMOLENS (Penel et al., 2009). Examples of PAH1 homologs include, but are not limited to, lipins in mammalians and some other vertebrates (encoded by *Lpin1, Lpin2,* and *Lpin3;* Gene ID: 23175, 9663, and 64900 in humans and 14245, 64898 and 64899 in mice, respectively), ned1 in *Schizosaccharomyces* (GeneID: 2542274), CG8709 in *Drosophila* (GeneID: 35790), AgaP_AGAP007636 in *Anopheles* (GeneID: 1269590), and AT3G09560 (GeneID: 820113) and AT5G42870 (GeneID: 834298) in *Arabidopsis thaliana.* A particularly envisaged PAH1 homolog is lipin-1. Typical of these PAH1 homologs is that they possess a NLIP domain with a conserved glycine residue at the N-terminus and a HAD-like domain with conserved aspartate residues in the catalytic sequence DIDGT (SEQ ID NO: 11) (Péterfy et al., 2001; Han et al., 2007; Carman and Han, 2009).

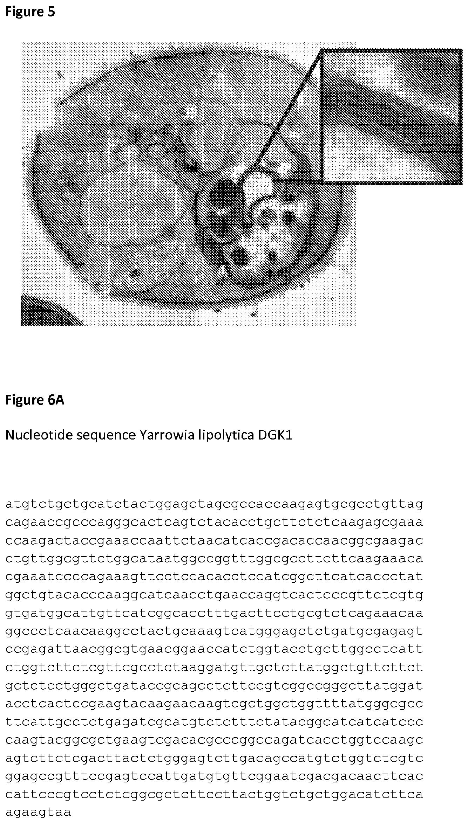

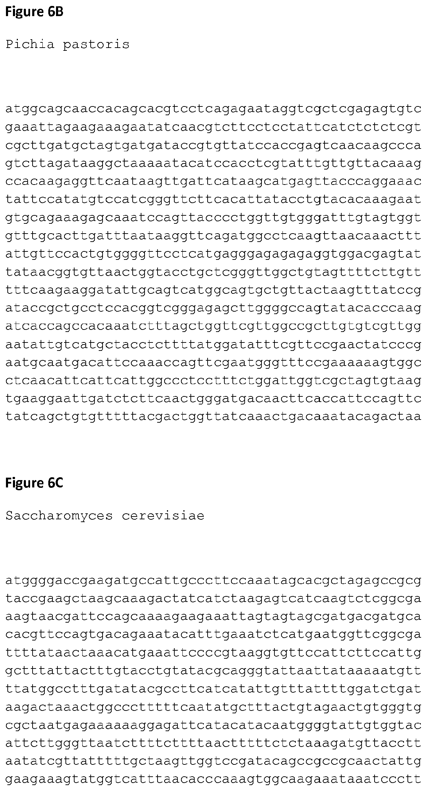

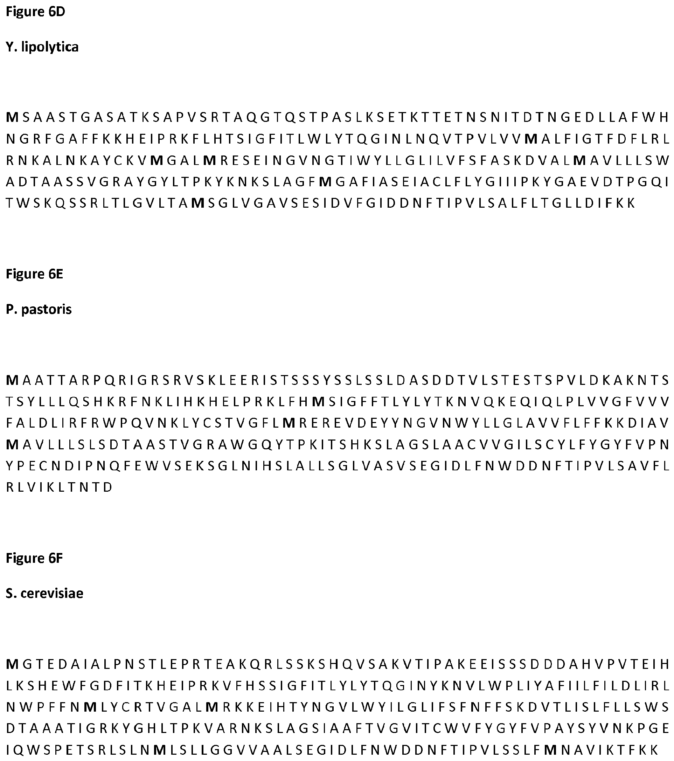

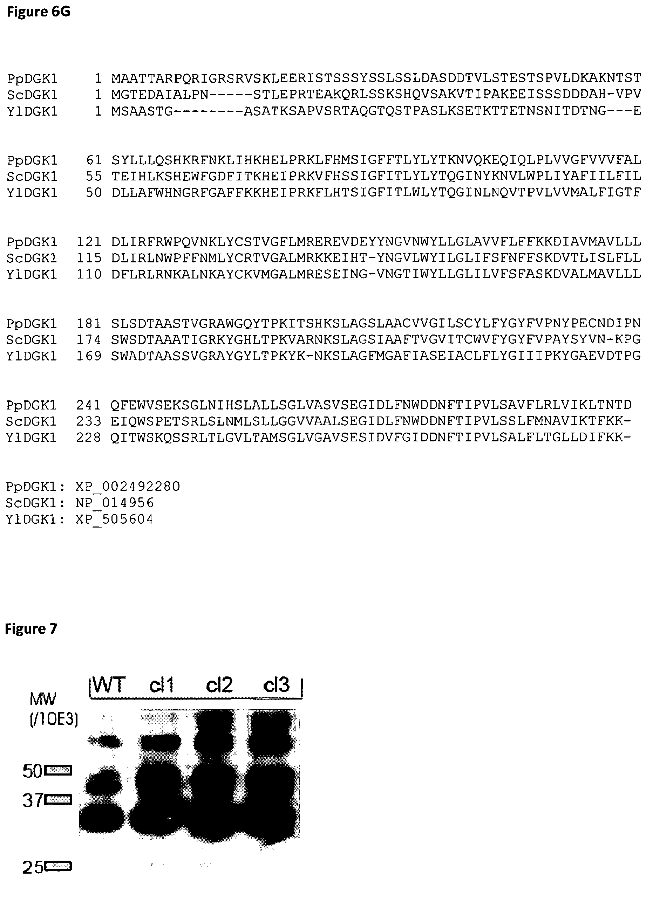

The term "diacylglycerol kinase", "DAGK" or "DGK" as used herein refers to an enzyme catalyzing the reverse reaction as a phosphatidic acid phosphatase, i.e. the phosphorylation of DAG to obtain phosphatidic acid (EC 2.7.1.107 for the ATP-dependent DGK; in yeast, the enzyme is CTP-dependent (Han et al., 2008a, 2008b) and EC 2.7.1.n5 has been proposed as nomenclature in the Uniprot database). The term "DGK1" as used herein refers to the yeast DGK enzyme and the encoding gene (GeneID: 854488 in *Saccharomyces cerevisiae;* Gene ID: 8199357 in *Pichia Pastoris* and Gene ID: 2909033 for *Yarrowia lipolytica.* The gene and protein sequences of these DGK1s are also shown in Figure 6, sometimes also indicated as HSD1.
A "DGK1 homolog" as used throughout the application refers to genes and proteins in species other than yeast homologous to DGK1 and having diacylglycerol kinase activity. Homology is as detailed above. DGK1 homologs are found throughout the eukaryotes, from yeast over plants (Katagiri et al., 1996; Vaultier et al., 2008) to *C*. *elegans* (Jose and Koelle, 2005) and mammalian cells (Sakane et al., 2007). Typically, DGK1 in yeast uses CTP as the phosphate donor in its reaction (Han et al., 2008b) while DGK1 homologs in e.g. mammalian cells use ATP instead of CTP (Sakane et al., 2007).

The term "inducible promoter" as used herein refers to a promoter that can be switched 'on' or 'off' (thereby regulating gene transcription) in response to external stimuli such as, but not limited to, temperature, pH, certain nutrients, specific cellular signals, etcetera. It is used to distinguish between a "constitutive promoter", by which a promoter is meant that is continuously switched 'on', i.e. from which gene transcription is constitutively active.

A "glyco-engineered yeast" as used herein is a yeast wherein the naturally occurring modifications on glycoproteins have been altered by genetic engineering of enzymes involved in the glycosylation pathway. A "glycoprotein" is a protein that carries at least one oligosaccharide chain. Typical monosaccharides that may be included in an oligosaccharide chain of a glycoprotein include, but are not limited to, glucose (Glu), galactose (Gal), mannose (Man), fucose (Fuc), N-acetylneuraminic acid (NeuAc) or another sialic acid, N-acetylglucosamine (GlcNAc), N-acetylgalactosamine (GalNAc), xylose (Xyl) and derivatives thereof (e.g. phosphoderivatives). Sugar chains may be branched or not, and may comprise one or more types of oligosaccharide. In general, sugar chains in N-linked glycosylation may be divided in three types: high-mannose, complex and hybrid type glycosylation. These terms are well known to the skilled person and defined in the literature. Briefly, high-mannose type glycosylation typically refers to oligosaccharide chains comprising two N-acetylglucosamines with (possibly many) mannose and/or mannosylphosphate residues (but typically no other monosaccharides).
Complex glycosylation typically refers to structures with typically one, two or more (e.g. up to six) outer branches with a sialyllactosamine sequence, most often linked to an inner core structure Man3GlcNAc2. For instance, a complex N-glycan may have at least one branch, or at least two, of alternating GlcNAc and galactose (Gal) residues that may terminate in a variety of oligosaccharides but typically will not terminate with a mannose residue.

Hybrid type glycosylation covers the intermediate forms, i.e. those glycosylated proteins carrying both mannose and non-mannose residues in addition to the two N-acetylglucosamine residues. In contrast to complex glycosylation, at least one branch of hybrid type glycosylation structures ends in a mannose residue.
Typically, but not necessarily, natural yeast will have a high mannose type glycosylation, and it can be glyco-engineered in part or in whole towards hybrid glycosylation or even complex glycosylation (to more closely resemble glycosylation found in mammalian cells). Examples of glyco-engineered yeast strains are known in the art (e.g. Wildt and Gerngross, 2005; Jacobs and Callewaert, 2009).

A "virus-like particle" or "VLP" as used herein consist of proteins that form a virus's outer shell and the surface proteins, without the genetic material required for replication. It thus forms a genetically engineered spherical protein envelope derived from a virus that does not contain viral genetic material and cannot replicate but can elicit an immune response and thus be used for vaccine purposes. VLPs have been produced from components of a wide variety of virus families including Parvoviridae (e.g. adeno-associated virus), Retroviridae (e.g. HIV), and Flaviviridae (e.g. Hepatitis C virus). Although VLPs may be entirely made up of proteins, a particular class of VLPs are lipid-containing VLPs (e.g. derived from viruses with a lipid envelope, such as influenza or herpes viruses) or lipoparticles (US 7,763,258), which contain lipids in their outer shell, which makes them particularly suitable for the expression of membrane proteins.

The present specification describes cells or cellular systems (cultures, organisms) that can express high concentrations of proteins, particularly membrane proteins. Also, methods are described that use such cells or cellular systems to produce higher amounts of such proteins than is feasible with existing methods.

The present specification describes eukaryotic cells deficient in expression and/or activity of an endogenous phosphatidic acid phosphatase, and/or overexpressing a diacylglycerol kinase. These cells may not be of the species *Saccharomyces cerevisiae.* The cells described may be further typically contain a nucleic acid sequence encoding a protein to be expressed in said cell. Most particularly, the nucleic acid sequence is an exogenous sequence or an endogenous sequence under control of an exogenous promoter. The cells may also be of the species *Saccharomyces cerevisiae.*
Accordingly, methods of enhancing protein production in such eukaryotic cells are described. These methods entail a eukaryotic cell deficient in expression and/or activity of an endogenous phosphatidic acid phosphatase, and/or overexpressing a diacylglycerol kinase, wherein the cell comprises a nucleic acid sequence encoding the protein of interest and the cell is maintained in conditions suitable for expressing the protein. Particularly where the eukaryotic cell is from the species *Saccharomyces cerevisiae,* the nucleic acid sequence encoding the protein of interest may be an exogenous sequence or an endogenous sequence under control of an exogenous promoter. This is envisaged for other species as well. The expressed protein may further optionally be isolated and/or purified.
The nature of the eukaryotic cells, both as such and as used in the methods described herein, can be very varied, since all eukaryotic cells have an endoplasmic reticulum and it is through expansion of this membrane that protein production is increased. Also, phosphatidic acid phosphatases and diacylglycerol kinases occur in all kinds of eukaryotic cells, and it has been shown that their function is evolutionarily conserved from unicellular eukaryotes to mammals (Grimsey et al., 2008). In this regard, it should be stressed that the technical effect of PAP inhibition is identical to that of increasing DGK activity, since the enzymes catalyze opposite directions of the same reaction.
It is particularly envisaged that the eukaryotic cells used are eukaryotic cells that are normally used as expression systems, to take further advantage of optimized protein production. Examples of eukaryotic cells that are used for protein production include, but are not limited to, yeast cells (e.g. *Pichia, Hansenula, Yarrowia),* insect cells (e.g. SF-9, SF-21, and High-Five cells), mammalian cells (e.g. Hek293, COS, CHO cells), plant cell cultures (e.g. *Nicotiana tabacum, Oryza sativa,* soy bean or tomato cultures, see for instance Hellwig et al., 2004; Huang et al., 2009), or even whole plants. The present specification describes that the cells may be provided as such, as a eukaryotic cell culture, or even as an organism (i.e. a non-human organism). However, the organism is not a mouse, or not even a mammal.

It is particularly envisaged that the eukaryotic cells are yeast cells, as these are very amenable to protein production and are robust expression systems. Even more particularly, the yeast cells may be methylotrophic yeast cells, such as species of the genus *Hansenula* (e.g. *Hansenula polymorpha*), species of the genus *Candida* (e.g. *Candida boidinii*) or most particularly species of the genus *Pichia,* such as *Pichia pastoris.* The yeast cells may be of the genus *Yarrowia,* most particularly of the species *Yarrowia lipolytica.* The specification describes that the yeast cells may not be from the species *Saccharomyces cerevisiae* or even not from the genus *Saccharomyces.* The yeast cells may be glyco-engineered yeast cells. Examples thereof include, but are not limited to, yeast cells that have humanized glycosylation patterns. Typically, the protein that is expressed in a yeast cell will be isolated (or possibly secreted) from the cell.

The present specification also describes that the eukaryotic cells may be plant cells, particularly plant cell cultures. The eukaryotic cells may also be mammalian cells, most particularly Hek293 cells, such as Hek293S cells.

To make a cell deficient in expression and/or activity of an endogenous phosphatidic acid phosphatase, several strategies can be used, and the nature of the strategy is not vital to the application, as long as it results in diminishing PAP activity to the extent that the endoplasmic reticulum is expanded in the cell. Cells can be made deficient for PAP at the genetic level, e.g. by deleting, mutating, replacing or otherwise disrupting the (endogenous) gene encoding PAP. Alternatively, one can interfere with transcription from the PAP gene, or remove or inhibit the transcribed (nucleic acid, mRNA) or translated (amino acid, protein) gene products. This may for instance be achieved through siRNA inhibition of the PAP mRNA. Also morpholinos, miRNAs, shRNA, LNA, small molecule inhibition or similar technologies may be used, as the skilled person will be aware of. The PAP protein can for instance be inhibited using inhibitory antibodies, antibody fragments, scFv, Fc or nanobodies, small molecules or peptides.
Interestingly, PAP activity may also be inhibited without directly interfering with PAP expression products. For instance, in yeast it has been shown that the loss of the dephosphorylated form of the yeast PAP enzyme PAH1 by deletion of Nem1 and/or Spo7, which form a complex that dephosphorylates PAH1, results in the same phenotype as deletion of PAH1 (Siniossoglou et al, 1998; Santos-Rosa et al., 2005). Likewise, increased phosphorylation of Lipin 1 and 2 inhibits their PA phosphatase activity (Grimsey et al., 2008). Thus, a cell may be made deficient in PAP activity by increasing PAP phosphorylation (or blocking PAP dephosphorylation).
As will be clear to those of skill in the art, deficiency of PAP expression and/or activity may both be constitutive (e.g. genetic deletion) or inducible (e.g. small molecule inhibition).
Typically, the endogenous phosphatidic acid phosphatase is PAH1 or a homolog thereof.

The eukaryotic cells described herein may, either solely or in addition to PAP deficiency, overexpress a diacylglycerol kinase. This may be endogenous DGK that is overexpressed, for instance by means of an exogenous promoter. The exogenous promoter may be constitutive or inducible, but typically will be a stronger promoter than the endogenous promoter, to ensure overexpression of DGK. Alternatively, an exogenous diacylglycerol kinase may be overexpressed, i.e. the eukaryotic cell may be genetically engineered so as to express a DGK that it does not normally express. The exogenous DGK may for instance also be a non-naturally occurring DGK, such as for instance a functional fragment of a diacylglycerol kinase. A fragment is considered functional if it retains the capability to catalyze the phosphorylation reaction of DAG to obtain phosphatidic acid. As for endogenous DGK, the exogenous DGK may also be under control of a constitutive or inducible promoter. The nature of the promoter is not vital to the application and will typically depend on the expression system (cell type) used and/or on the amount of protein that is needed or feasible. It is described that the diacylglycerol kinase that is overexpressed may be DGK1 or a homolog thereof.

Particularly envisaged are cells that combine a deficiency in endogenous PAP with overexpression of a DGK, for even higher production of proteins, although the effect is not necessarily additive. Note that the cells described herein that are characterized by significant membrane expansion (by having a PAP deficiency and/or overexpressing a DGK) may be further engineered for increased protein expression. A non-limiting example thereof is overexpression of HAC1 (Guerfal et al., 2010), but other modifications are also known in the art. Alternatively or additionally, the cells may be further engineered to perform eukaryotic post-translational modifications (e.g. De Pourcq et al., 2010).

The proteins that are produced in the cells described herein will typically be encoded by an exogenous nucleic acid sequence or an endogenous nucleic acid sequence under control of an exogenous promoter, i.e. the cells are engineered to express the protein of interest. The protein may be expressed constitutively or in an inducible way. Accordingly, the promoter may be a constitutive or inducible promoter.

The present specification describes that the proteins that are produced in the eukaryotic cells described herein may be membrane-bound proteins. The protein may be a receptor. The protein may be a GPCR. Examples of GPCRs envisaged for production using the methods described herein include, but are not limited to, Calcium-sensing receptor, CXCR4, endothelin receptor B, follicle stimulating hormone receptor, N-formyl peptide receptor, frizzled receptor (FZD4), gonadotropin-releasing hormone receptor, GPR54, GPR56, Kaposi's sarcoma constitutively active GPCR, melanocortin 4 receptor, rhodopsin, vasopressin receptor, β1 adrenergic receptor, β2 adrenergic receptor, β3 adrenergic receptor, α adrenergic receptors, CCR2, CCR5, dopamine receptor, dopamine receptor 2, dopamine receptor 3, muscarinic receptor subtype 3, GPR154, P2Y₁₂, 5-HT receptors, angiotensin II receptors, adenosine A2AR, substance PNK1R, serotonin receptors (e.g. 5HT1D), histamine receptors (e.g. HH1R), opioid receptors (e.g. OPRK), and parathyroid hormone receptor 1 (see e.g. Insel et al., 2007 for a review of some of these).
The methods for GPCR expression described herein can also be combined with known improvements for GPCR expression that typically involve production of GPCR variants. Examples thereof include, but are not limited to, the use of a signal sequence specific to the species of eukaryotic cell used rather than the GPCR-specific signal sequence, the use of a C-truncated GPCR versus the use of an intact GPCR, the use of a GPCR with a sequence insertion (e.g. a T4 lysozyme coding sequence) in the 3^{rd} loop, and so on. Of course, these different variations can further be combined with each other.

Apart from receptors, the cells and methods may also be used to improve production of secreted proteins, which are also synthesized in the ER. The increased membrane surface (and enclosed volume) indeed allows for more proteins to be synthesized and secreted.

The present specification further describes that the produced proteins are functional, for instance produced receptors remain capable of ligand binding and signal transduction.

The present specification also describes that more than one, i.e. two or more different proteins may be produced simultaneously. The proteins may all be membrane-bound, all be secreted proteins or a mixture thereof. When more than one protein is produced, care will typically be taken that they can be recovered easily either separately or together. It is described that even higher production can be achieved by expressing multiple copies of the protein to be expressed, e.g. as a polyprotein.

A special case of proteins that are envisaged to be produced together are proteins that form supramolecular entities. One such example are structural proteins, e.g. structural proteins making up a virus-like particle (VLP). VLPs mimic the overall structure of virus particles without any requirement that they contain infectious genetic material, and thus are ideally suited as a highly effective type of subunit vaccines. Indeed, many VLPs lack the DNA or RNA genome of the virus altogether, but have the authentic conformation of viral capsid proteins seen with attenuated virus vaccines, without any of the risks associated with virus replication or inactivation. VLP preparations are all based on the observation that expression of the capsid proteins of many viruses leads to the spontaneous assembly of particles that are structurally similar to authentic virus (Miyanohara et al., 1986; Delchambre et al., 1989; Gheysen et al., 1989; French et al., 1990). Moreover, VLPs have been successfully produced in many expression systems, such as yeast and insect cell systems. Interestingly, many VLPs bud from the internal (ER-derived) membranes. As it is feasible to make VLPs from viruses with lipid envelopes which are derived from the host cell (such as influenza, HIV and HCV; Roy and Noad, 2008), the enhanced membrane formation seen in the cells provided herein can serve to increase VLP production in the cells. This holds true for VLPs budding from ER-derived membranes, but also for VLPs budding from the plasma membrane, as membrane proteins are synthesized in ER prior to being transported to the plasma membrane.

The present specification describes that where the expression of viral structural proteins, such as Envelope or Capsid, is envisaged for self-assembly of VLPs, the cells may comprise one or more nucleic acid sequences encoding the one or more proteins making up the virus-like particle, in conditions suitable for expressing the one or more proteins. Where the VLP consists of more than one protein, the different proteins can also be expressed as a single polyprotein (Bräutigam et al., 1993; Kibenge et al., 1999). As mentioned, VLPs can be generated for a whole variety of viruses, infecting humans, animals or even plants. These include, but are not limited to, HIV, respiratory syncytial virus (RSV), hepatitis B virus, hepatitis C virus, hepatitis E virus, Epstein Barr virus, HPV, measles virus, influenza virus, influenza A virus, Norwalk and Norwalk-like virus, feline calicivirus, porcine parvovirus, mink enteritits parvovirus, canine parvovirus, B19, AAV, chicken anemia virus, porcine circovirus, SV40, JC virus, murine polyomavirus, polio virus, bluetongue virus, rotavirus, SIV, FIV, Visna virus, feline leukemia virus, BLV, Rous sarcoma virus, Newcastle disease virus, SARS coronavirus, Hantaan virus, infectious Bursal disease virus, Dengue virus, and encephalitis virus (see e.g. Noad and Roy, 2003; Roy and Noad, 2008; Sugrue et al., 1997; Falcon et al., 1999; Liu et al., 2010).

During or after the protein production in the eukaryotic cells, the protein or proteins of interest can be recovered from the cells. Accordingly, the methods of protein production may optionally also comprise the step of isolating the expressed protein. This typically involves recovery of the material wherein the protein is present (e.g. a cell lysate or specific fraction thereof, the medium wherein the protein is secreted) and subsequent purification of the protein. Means that may be employed to this end are known to the skilled person and include specific antibodies, tags fused to the proteins, affinity purification columns, and the like.

It is to be understood that although particular embodiments, specific configurations as well as materials and/or molecules, have been discussed herein for cells and methods according to the present invention, various changes or modifications in form and detail may be made without departing from the scope of this invention. The following examples are provided to better illustrate particular embodiments, and they should not be considered limiting the application. The application is limited only by the claims.

### Examples

### Example 1. Identification and knock-out of PAH1 in Yarrowia lipolytica.

It is shown that the knock out of a single gene *PAH1* in the yeast *Yarrowia lipolytica* can lead up to a 10-fold higher expression of eukaryotic membrane proteins. *PAH1* has been identified in *S*. *cerevisiae* as a protein that couples phospholipid synthesis to growth of the nuclear/endoplasmic reticulum membrane (Santos-Rosa et al., 2005). Mutants defective in the *PAH1* gene show a massive nuclear/ER membrane expansion.

The sequence of the *PAH1* gene in *Yarrowia lipolytica* was found through a homology search of the *Saccharomyces cerevisiae PAH1* (GeneID: 855201) gene against the genome of *Y. lipolytica* using the Basic Local Alignment search Tool (Blast) from ncbi. The blast revealed the protein YALI0D27016g (Fig. 1), which shows some similarities with the nuclear elongation and deformation protein 1 from *Schizosaccharomyces pombe.* Analysis of the sequence shows the presence of the conserved haloacid dehalogenase (HAD)-like domain in the middle of the protein sequence which contains a DxDxT motif (Han et al., 2007). Also the terminal N-terminal lipin domain, characteristic for the *PAH1* gene, could be identified (Fig. 1B).

A *PAH1* knock out construct was generated on leu2 as selection marker and transformed to 4 clones expressing a different amount of 5HT1D expression cassettes. The gene construction strategy used to knock out the *PAH1* gene is described in the material and methods section. The gene was disrupted by double homologous recombination after transformation of the linearized knock out fragment to the respective strains. Clones phototrophic for leucine were screened with PCR on genomic DNA with the primers pah1ylPfw07-010: 5'GCGGCCGCGAAGACGGTGAGTATGGCCATC 3' (SEQ ID NO: 12) and pah1ylTrv07-007: 5' GCGGCCGCCCAAACCATGCATACAAATCAG 3' (SEQ ID NO: 13). Positive identified clones by PCR were confirmed by Southern blot. Knock outs do not show the wild type fragment of 758 bp but a band of 1253 bp. Random integrants show bands of both sizes.

To analyze the expression levels of the 5HT1D receptor of the knock out strains versus the wild type strains, an expression experiment was performed. Cells were induced on oleic acid for 24 hours and then harvested. Before and after induction the optical density was measured. It could clearly be seen that the *PAH1* knock out strains show a slower growth compared to the WT strains (Fig. 3).

To evaluate the expression levels of the receptor, cells were broken with glass beads and membrane fractions were isolated. A BCA protein assay was done to determine the amount of total membrane protein and a same amount of the proteins was loaded on a 12% SDS-PAGE gel. After separation of the proteins, proteins were blotted on to a nitrocellulose membrane (PerkinElmer). Immunoblotting shows an at least ten fold higher expression of the receptor compared to the wild type strains (Fig. 4).

Electron microscopy was performed in order to obtain a phenotypical analysis of the *PAH1* knock out strains. On the TEM pictures it could be seen that the knock out strain shows a region of stacked membranes which are continuous with the outer nuclear membrane and hence the ER membrane (Fig. 5). These stacked membranes were also reported in *the S.cerevisiae* knock out strain (Santos-Rosa et al., 2005).

### Example 2. Isolation and cloning of the diacylglycerol kinase 1 (DGK1) of Pichia pastoris.

Recently an enzyme counteracting the phosphatase activity of PAH1 was identified in *S*. *cerevisiae* (Han et al., 2008a and b). It is reported that overexpression of DGK1 leads to an accumulation of PA and consequently expansion of the nuclear/ER membrane as seen in the *Δpah1* mutant. Unlike hydroxymethylglutaryl-Co-A and cytochrome b5 which induce membrane expansion in the form of karmellae also when the catalytically inactive enzymes are overexpressed, membrane expansion is only observed when active dgk1p is overexpressed (Han et al., 2008b).

The DGK1 sequence of P. pastoris was found through a homology search of the S. cerevisiae DGK1 against the P. pastoris genome. Primers were ordered to isolate the DGK1 from the genome (DGK1pp09-007 (5'-AAAAACAACTAATTATTCGAAATGGCAGCAACCACAGCACGTCC-3' (SEQ ID NO: 14)) and DGK1pp09-008 (5'-AAGGCGAATTAATTCGCGGCCGCTTAGTCTGTATTTGTCAGTTTG-3' (SEQ ID NO: 15))). After isolation the gene was cloned in the pPIChyg vector resulting in the plasmid pPIChygDGK1 using the CloneEZ kit from GenScript. The vector was linearized in the AOX1 promotor with the restriction enzyme Pme1 and transformed via electroporation to a P. pastoris strain expressing the Adenosine A2A receptor under control of the AOX1 promotor. Integration of the plasmid was confirmed through PCR on genomic DNA.

Positive clones were evaluated on the expression of the A2A receptor. Clones were grown for 48 h on BMGY, washed once with BMY and then resuspended in BMMY (1% methanol). Methanol was added every 12 hours to sustain protein induction. After induction cells were harvested and evaluated for receptor expression through western blot using the Rho1D4 tag present on the protein. From the Western Blot it is seen that the dgk1p overexpressing strains show a higher expression of the receptor (Figure 7).

### Growth of the dgk1p overexpressing strain.

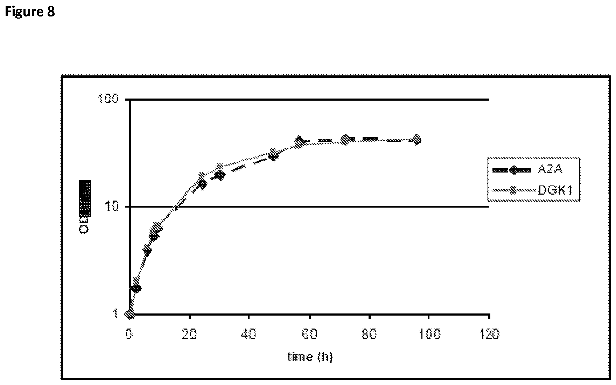

As it is known that the *S*. *cerevisae Δpah1* show a growth defect (Santos-Rosa et al., 2005), we evaluated if the dgk1p overexpressing strain also shows slower growth at 30°C. A preculture was grown in BMGY, OD600 was measured and cells were diluted to an OD600 of 1 in BMMY. However, no slower growth was observed (Figure 8).

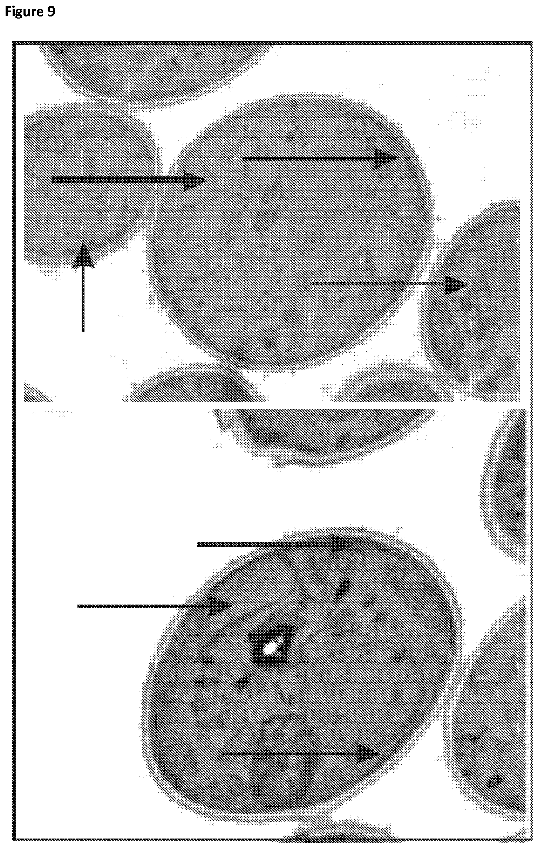

Membrane expansion of the engineered dgk1p overexpressing strain was evaluated by electron microscopy after 24h of induction of the dgk1p. It can be seen that the membrane phenotype is similar to the PAH1 knock-out (Figure 9), as was previously reported for S. cerevisiae (Han et al., 2008a,b).

### Example 3. Disruption of the PAH1 gene of P. pastoris.

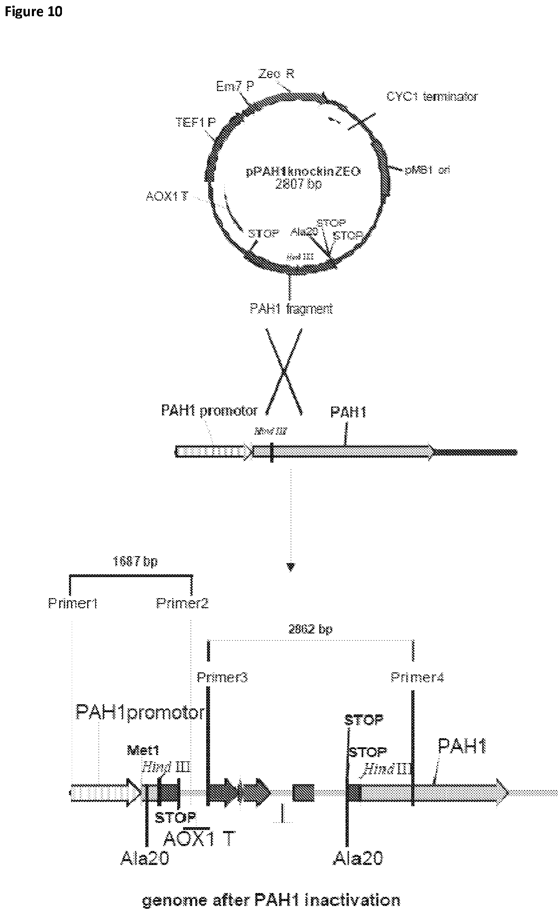

A knock out strain of the *PAH1* gene in *P. pastoris* was created. As double homologous recombination at this locus seems to occur very rarely, it was decided to create a disruption cassette to disrupt the gene rather than to knock out the gene. The *PAH1* gene of a *P. pastoris* expressing the Adenosine A2A receptor was disrupted after transformation of the plasmid pPAH1knockinZEO. Positive clones were identified by PCR. The cloning strategy is shown in Figure 10. Briefly, after digestion of the plasmid pPAHlknockinzeo with Hindlll and transformation, the plasmid integrates in the PAH1 locus. Integration of the vector results in a short fragment not containing the catalytic active domain of the PAH1 gene and a fragment that cannot be translated because of the absence of a promoter and the presence of 2 in frame nonsense codons. Integration of the plasmid was confirmed via PCR. A first PCR (primer 1 and primer 2) results in a fragment of 1687 bp and a second PCR results (primer 3 and primer 4) in a fragment of 2862 bp. PCR fragments were isolated and sequenced to confirm correct integration.

### Growth analysis of PAH1 disruption clone

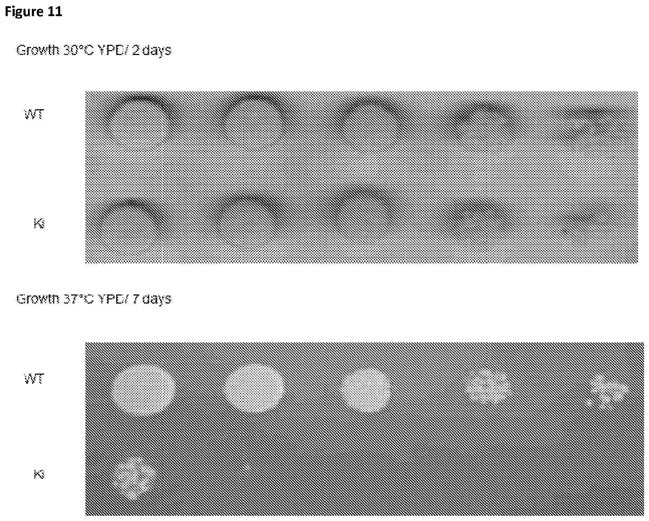

Growth at 30°C and 37°C was evaluated. As expected, a slower growth was observed at 37°C in agreement with the temperature sensitivity seen for the *Δpah1* mutant of *S*. *cerevisae.* At 30°C no growth defect is seen comparable with the observations of the dgk1p overexpression (Example 2) (Figure 11).

### Evaluation of the membrane expansion in the PAH1 disruption clone

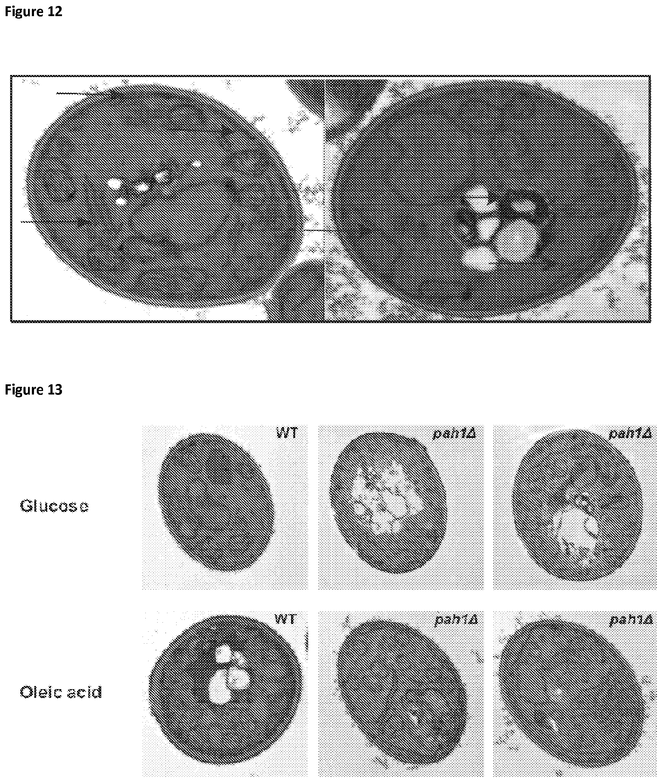

Membrane expansion was evaluated through EM (Figure 12). Here also, extra membranes are observed. The membranes of these yeast cells appear often closely associated with phagosomes. These data suggest that the membranes might be eliminated over time. When *P. pastoris* cells are grown on oleic acid instead of glucose, the ER membrane increase is more pronounced, and not associated with vacuoles (Fig. 13). Thus, a strategy to increase the effect of the PAH1 KO on membrane protein expression might be to express the receptor from a fatty acid inducible promoter.

### Evaluation of expression levels of A2AR in the PAH1 disruption clone

The expression levels of the receptor A2AR were evaluated after 24 hours of induction on methanol containing medium on Western Blot. The obtained expression levels are comparable with the levels obtained in the dgk1p overexpressing strain and slightly higher than the WT strain (Figure 14A).

### Example 4. Functionality of expressed proteins

To test whether the GPCR produced in the mutant *Pichia pastoris* strains is also functional, ligand binding assays were performed on the expressed adenosine 2A receptor. The results are shown in Figure 14B. The ligand binding of the A2AR expressed in the WT, PAH1 and DGK1 strain was evaluated by a radioligand binding saturation assay. The 3 strains show a comparable ligand binding and the produced GPCR thus is functional. Although it may seem surprising that the higher expression level is not correlated to an increase in active binding receptor, this may be explained by the already high expression levels of A2AR in the wild type *Pichia* strain: a protein that expresses lower quantities in the wild type strain will likely result in different activities.

### Example 5: Evaluation of the expression level of the A2A receptor in a PAH1 knock out Yarrowia lipolytica strain

A PAH1 knock out strain was created in a PO1D(Leu-) strain overexpressing the Adenosine A2A receptor. The knock out strategy is described in the material and methods section. Expression levels of the receptor were analyzed on a Western Blot (shown in Figure 15A) and ligand binding of the receptor was determined by radioligand binding assays (Fig. 15B). The PAH1 knock out strains shows higher expression levels of the receptor. These do not strictly correlate with the ligand binding data. As mentioned above, this may be due to the already high expression levels of A2AR and thus maximum functionality. Additionally, in this case oleic acid was used to induce the cells, which may have altered the lipid environment and rendered part of the produced receptors conformationally inactive. This problem is easy to overcome by choosing a different induction strategy that does not influence the membrane lipid environment.

The experiment was repeated using cells grown on oleate and glucose as a carbon source. Because it is described that a *PAH1* deletion strain of *S*. *cerevisiae* has a slower growth, we analyzed the growth of a *Y. lipolytica ΔPAH1* strain. From Figure 16 it can be seen that cells exhibited a different growth pattern dependent on the carbon source. Only a slight growth retardation is seen on glucose, especially in the early exponential phase. Cells grown on oleic acid show a remarkable longer lag phase but achieve the stationary phase at a same optical density as the wild type strain. Indeed it has been reported that *S*. *cerevisae* strains defective in neutral lipid synthesis (ΔARE1, ΔARE2, ΔDGA1, ΔLRO1) show an extended lag phase but can adapt to oleate growth. The authors speculate that this is due to the activation of gain-of-function suppressor genes (Connerth et al., 2010).

When achieving protein production, it was again observed that the deletion strain displays much higher protein production than the wild type (WT) strains and this as well on oleic acid as glucose. The increase on oleic acid is higher than on glucose (Figure 18). This might be explained by the higher membrane expansion seen in these cells as can be seen from electron microscopy pictures taken at the end of protein induction (Figure 17). One should also keep in mind that the initial expression levels for the strains expressing the A2A receptor from the hp4d promoter is higher than for the POX2 promoter.

Big lipid droplets are observed in the WT strain grown on oleic acid while the ΔPAH1strains has none or very little and small lipid droplets, which is consistent with its defect in neutral lipid biosynthesis. The ER membrane proliferation in the strain grown on oleic acid medium is massive (Fig. 17).

### Example 6. Production of VLPs, budding from the endoplasmic reticulum, which contain a protein(s) of interest.

Here we describe the production of VLPs which bud from the internal membranes, more specifically from the ER. As the elevated PA content (resulting from PAP inhibition and/or increase in DGK activity) results in the increase of internal membrane, more VLPs will be able to bud from the ER of this engineered strain. One example is the expression of Hepatitis B surface antigen (HBsAg) from Hepatitis B virus (HBV). Particles containing HBsAg purified from plasma of asymptomatic HBV carriers, have already been used for vaccination since the early eighties. However, this type of vaccines is not frequently used for mass immunization due to safety concerns, restricted supply and costs.

It has already been shown by others (Gurramkonda et al., 2009; Hadiji-Abbes et al., 2009) that the overexpression of HBsAg alone is sufficient to produce ER-derived intracellular VLPs in *Pichia pastoris.* Upon co-expression of HBsAG from the Hepatitis B Virus (HBV) and a protein of interest in yeast, VLPs containing the protein of interest can be obtained.

### Cloning of HBsAg of the Hepatitis B Virus.

The full-length sequence of HBsAg is obtained as a synthetic gene and amplified by PCR. The gene is cloned in a vector containing the zeocin resistance marker using the BstBI and NotI restriction sites. The vector is linearized in the AOX1 promoter with Pmel and transformed via electroporation to *P. pastoris.* Integration of the plasmid is confirmed through PCR on genomic DNA.

### Production and purification of HBsAg-based VLPs.

Clones are grown to saturation (± 48h) on BMGY at 30°C, collected by low-speed centrifugation and washed once with 1 x PBS (Phosphate-Buffered Saline). They are then transferred to BMMY (1% methanol) for another 1-24h at 30°C. Different lengths of induction times are possible, induction can be performed before or after spheroplast formation. Methanol is added every 12h to sustain protein induction.

The yeast cells are ruptured upon heavy vortexing with glass beads for 5 times 3 minutes at 4°C. The debris is discarded from the lysate after centrifugation at 2,000 rpm for 20 minutes at 4°C. The supernatant is transferred to a clean microcentrifuge tube and BCA-assay is performed to estimate the total protein concentration.

### Evaluation of HBsAg-based VLPs.

The supernatant is analyzed by SDS-PAGE and subsequent western blotting using rabbit anti-HBsAg antiserum and anti-rabbit IgG-HRP for visualization. Quantitation is performed by Coommassie brilliant blue staining and western blot on the supernatant, compared to serial dilutions of recombinant soluble HBsAg protein. An antibody against the protein of interest can be used to evaluate expression of this protein.

The production of VLPs is analyzed by electron microscopy on purified HBsAg as described previously by O' Keefe and Paiva, 1995; and Gurramkonda et al., 2009.

### Example 7. Production of VLPs, budding from the plasma membrane, which contain adenosine A₂A receptor

Here we describe the production of VLPs which bud from the plasma membrane. As the elevated PA content (resulting from PAP inhibition and/or increase in DGK activity) results in the increase of internal membrane, more membrane protein-containing VLPs will be formed. We describe the production of VLPs containing adenosine A₂A receptor as a protein of interest upon co-expression of the major structural protein Gag from the Human Immunodeficiency Virus (HIV) type-1 and the adenosine A₂A receptor in yeast.

It has been shown that the expression of Gag protein alone is sufficient to produce Gag VLPs that are morphologically identical to the immature form of HIV particles (Smith et al., 1993). The VLPs bud from the plasma membrane of eukaryotic cells when a large multimer is formed, resulting in a Gag-protein core enclosed by a plasma membrane-derived lipid bilayer (Tritel and Resh, 2000). This plasma membrane can be enriched for the adenosine A₂A receptor (or, if desired, other protein(s) of interest) by recombinant overexpression. Without organelle-specific targeting signals, a membrane protein (MP) or membrane-associated protein (MAP) is transported to the plasma membrane from which VLPs can bud. These VLPs can then be used for all kinds of applications: vaccines, structural biology (crystallography, NMR,...), drug screening, ligand binding, panning of antibody/nanobody libraries...

### Cloning of GAG of the Human Immunodeficiency Virus type-1.

The full-length sequence of Gag from HIV-1 is obtained as a synthetic gene and amplified by PCR using a forward primer, 5'-TTCGAAATGGGTGCGAGAGCGTCAGT-3' (SEQ ID NO: 16), and a reverse primer, 5'-GCGGCCGCTTATTGTGACGAGGGGTC-3' (SEQ ID NO: 17). The gene is cloned in a vector containing the zeocin resistance marker using the BstBI and NotI restriction sites. The vector is linearized in the AOX1 promoter with the restriction enzyme Pmel and transformed via electroporation to a *P. pastoris* strain expressing the Adenosine A₂A receptor under control of the AOX1 promoter. Integration of the plasmid is confirmed through PCR on genomic DNA.

### Production and purification of Gag-based VLPs.

Positive clones are evaluated in first instance for the presence of VLPs. Clones are grown to saturation (± 48h) on BMGY at 30°C, collected by low-speed centrifugation and washed once with wash buffer (50mM Tris, pH7.5, 5mM MgCl2, 1M sorbitol). The cells are resuspended in wash buffer containing 30mM DTT and incubated at 30°C for 20 min with gentle agitation. The cells are then transferred to wash buffer containing 3mM DTT and Zymolyase-100T (AMS Biotechnology, UK) at a final concentration of 0.4mg/ml. The reaction is allowed to proceed at 30°C for 20 min with gentle agitation. After being washed twice with 1M sorbitol, spheroplasts are cultured at 30°C for another 1-24h in BMMY (1% methanol). Different lengths of induction times are possible, induction can be performed before or after spheroplast formation. Methanol is added every 12h to sustain protein induction.

The culture medium of the yeast spheroplasts, in which the VLPs are secreted, is clarified by low-speed centrifugation and then centrifuged through 30% (w/v) sucrose cushions at 4°C at 26,000 rpm for 90 min. The VLP pellet is resuspended with 1 x PBS (Phosphate-Buffered Saline) and centrifuged on 20-70% (w/v) sucrose gradient at 4°C at 35,000 rpm overnight. Purified VLPs are obtained by fractionation of the gradient (Protocol after Sakuragi et al., 2002).

### Evaluation of Gag-based VLPs.

The fractions are analyzed by SDS-PAGE and subsequent western blotting using anti-Gag mAb for Gag, anti-Rho1D4 mAb for the adenosine A2A receptor, and anti-mouse IgG-HRP for visualization. Quantitation is performed by Coommassie brilliant blue staining and western blot on the different purified VLP-fractions, compared to serial dilutions of recombinant soluble Gag protein and adenosine A₂A receptor.

### Material and methods

**Strains culture conditions and reagents:** *Escherichia coli* strains MC1061 were used for the amplification of recombinant plasmid DNA and grown in a thermal shaker at 37°C in Luria-Broth (LB) medium supplemented with 50 µg/ml of kanamycin. *Yarrowia lipolytica* PO1D *(Ieu2*/*Ura3)* strain transformed with the plasmid 5HT1D(POX/URA) and JMP62-5HT1D. Plasmids bearing the 5HT1D receptor respectively with and without lip2 prepro secretion signal. Four clones expressing a different amount of expression cassettes of the 5HT1D receptor were evaluated. Pichia pastoris strain GS115 (*his4*) transformed with the plasmid pPIC92A2A. Po1dΔOCH1 (MatA, leu2-270, ura3-302, xpr2-322, Δoch1). Yeast were standard grown in YPD (1% yeast extract/2% Peptone/2% dextrose).

**Small scale protein induction:** Induction experiments were performed in a 125 ml baffled flask with 12.5 ml culture. For all experiments a preculture was grown and cells were inoculated at an OD 0.1. The A2A receptor expressed from the hp4 promoter was grown for 24 hours in YTG (1% yeast extract/2% Tryptone/2% dextrose). For induction on oleic acid, cells were grown for 24 hours in YTG, washed once with water and than resuspended in induction medium (50 mM Phosphate buffer pH 6.8/ 1% yeast extract/ 2% Tryptone/ 2% oleic acid). Cells were grown for 24 hours before harvesting.

**Construction of the knock out plasmid and strains:** A PAH1 knock out construct was made according to Fickers et al (Fickers et al., 2003). In short, a 549 bp promoter (primer sequence: pah1ylPfw07-010: 5'GCGGCCGCGAAGACGGTGAGTATGGCCATC 3' (SEQ ID NO: 12) and pah1ylPrv07-006: 5'TAGGGATAACAGGGTAATCCATTGACACAGAACTCGACCT 3' (SEQ ID NO: 18)) and a 551 bp terminator fragment (primer sequence: pahlylTfw07-011: 5' ATTACCCTGTTATCCCTACCGTACTGTACACCGTAGTTTG 3' (SEQ ID NO: 19) and pah1ylTrv07-007: 5' GCGGCCGCCCAAACCATGCATACAAATCAG 3' (SEQ ID NO: 13)) were amplified from genomic DNA using PCR. DNA was amplified with Phusion polymerase (Finnzymes) The reverse primer of the promoter fragment and the forward primer of the terminator fragment contain the rare meganuclease *I-SceI* recognition site which can be used in a fusion PCR to obtain a PT fragment. (Promotor I-SceI and I-SceI terminator resulting fragments are then pooled and used for amplification of the Promotor-I-SceI-Terminator cassette). This fragment is then cloned as a blunt-ended fragment into the pCR-Blunt II-TOPO vector (Invitrogen) resulting in a pCR-Blunt-TOPO PT vector. For the construction of the final Promotor-selection marker-Terminator fragment, the plasmid was digested with the *I-SceI* meganuclease enzyme for rescue of the marker. In a next step the marker is cloned into the pCR-Blunt-TOPO PT vector after *I-SceI* digestion. Linearization by a *Not*I digest of the final plasmid, results in the final disruption cassette containing a promoter-leucine marker-terminator fragment. Disruption of the *PAH1* gene occurs by double homogenous recombination.

**Construction of the Pichia pastoris PAH1 disruption plasmid pPAHlknockinzeo:** A fragment of 151 AA (amino acids) from Ala20 to Thr151 was isolated via PCR with primers PAH1pp09-023 (5'-agatcttaataagccacactgagtggtgctattg-3') (SEQ ID NO: 20) and PAH1pp08-02 (5'-**GCGGCCGC**TTACGTATTCTCAGGACTTGAGCTCAC-3' (SEQ ID NO: 21)) introducing a BglII and NotI site respectively for cloning. The fragment was cloned in the pGlycoSwitchM8 plasmid (Vervecken et al applied and environmental microbiology 2004) by replacing the Δoch1 disruption sequence.

**Transformation:** Transformation of competent cells of *Yarrowia lipolytica* was performed as described in Boisramé et al., 1996.
Pichia pastoris cells were transformed according to the protocol from the *Pichia* Expression kit (Invitrogen Cat. No.K1710-01).

**Southern Blot:** Genomic DNA was prepared using the Epicenter Kit (Epicenter Biotechnologies, Madison, WI). Southern was performed according to the kit insert from Amersham Gene Images AlkPhos Direct labeling and Detection System. The probe was an *I-Sce*I/*Ava*I fragment from the vector pah1PLTpah1inverse. 15 µg DNA was loaded on a 1% agarose gel and blotted overnight to a Hybond-N+ membrane (Amersham) in 10 SSC. After blotting, the membrane was cross-linked to the membrane with a UV-cross linker.

### Induction

*Yarrowia lipolytica:* From a fresh plate a single colony was inoculated in 12.5 ml YTD (1% yeast extract/ 2% trypton/ 2% dextrose) in a 125 ml Erlenmeyer and grown for 24 hours at 28°C and 250 rpm. After 24 hours, cells were washed once with Sodium-phosphate buffer pH 6.8 and resuspended in induction medium containing 4% oleic acid (Sodium phosphate buffer pH 6.8/ 1% yeast extract/2% Tryptone/ 5% oleic acid). Induction was performed for 24 hours at 28°C and 250 rpm. At the end of the induction OD600 was measured. To avoid interference of not used oleic acid; the pellet was washed twice with UP water before OD measuring.
*Pichia pastoris*: Cells were inoculated in a total of 12.5 ml BMGY in a 125 ml shake flask and grown for 48 h. Cells were washed once with BMY, resuspended in BMMY, and induced for 48 h. Methanol (100%) was added every 8-12 h to a final concentration of 1% to maintain the induction. The adenosine A2A receptor was induced for 24 h before harvesting for analysis.

**Growth curve:** Cultures were grown overnight to saturation in YPD and diluted next day to an OD600 of 0.2. Incubation was continued and OD600 was measured at different time points. For the cultures grown in medium containing oleic acid cells were spun down for 10 min at 13,000 rpm and washed once with 0.1 N NaOH in order to avoid interference of oleic acid on the OD600 measurement. OD600 of different cultures in different media were measured in triplicate.

**Membrane preparation:** Membranes were prepared from cells that have been induced for 24-27 h. Cells were pelleted by centrifugation at 1500 xg and the pellet was resuspended in ice-cold breaking buffer (50 mM sodium phosphate buffer pH 7.4, complete protease inhibitor Roche/ 5% glycerol). Cells were then broken by vigorous vortexing with glass beads in a mixer mill for 10 times 1 min or 5 times 2 min at 4°C. Cells were separated from the membrane suspension by low speed centrifugation (2000 rpm, 5 min, 4°C or 1000xg, 30 min, 4°C). Membranes were pelleted at 100,000g and 4°C for 60 min and resuspended in resuspension buffer (50 mM sodium phosphate buffer pH 7.4/2%SDS supplemented with a complete protease inhibitor from Roche) and snap-frozen in liquid nitrogen until further testing. The protein concentration of the membrane preparation was determined using the BCA reagent (Pierce, Rockford,IL) with BSA as standard. Ten micrograms of total membrane protein was analyzed by western blot. The blot was blocked overnight in blocking buffer (0.05% Tween-20 and 3% casein in 1x PBS) and probed with a 1/500 diluted primary mouse anti-Rho1D4 antibody, followed by a 1/3000 diluted secondary anti-mouse IgG peroxidase from sheep (Sigma Cat.n°NA931V). Protein bands were visualized with Renaissance western blot chemiluminescence reagent plus (PerkinElmer).

**Protein analysis:** For each strain an equal amount of protein was dissolved in laemli buffer and heated for 10 min at 50°C. Samples were then loaded on a 12% SDS-PAGE. After electrophoreses samples were transferred to a nylon membrane (PerkinElmer). The blot was blocked overnight in blocking buffer (Tween-20 0.05%/3% casein/1xPBS) and probed with a 1/100 diluted primary mouse anti-Rho1D4 antibody followed by a 1/3000 diluted secondary anti-mouse IgG peroxidase from sheep (Sigma Cat.n°NA931V). Protein bands were visualized with Renaissance Western blot chemiluminescence reagent plus (PerkinElmer).

**Binding assays:** Radioligand binding assays were performed according to Weiss et al (Eur J Biochem 2002, 269(1):82-92). Briefly, 5 µg of total membrane protein was incubated with different concentrations (0.06-14 nM) of the A2AR antagonist ³[H]ZM241385 in 500 µl binding buffer (20 mM HEPES pH 7.4, 100 mM NaCl). Adenosine deaminase (0.1 U) was added to degrade the adenosine released from the membranes and the membranes were incubated at 22°C for 1 h. Non-specific binding was determined in the presence of 10 mM theophylline. After incubation, bound and free ligand were separated on Whatmann GF/B filters pretreated with 0.1% polyethylenimine using a Brandel cell harvester. The filters were washed three times with binding buffer and the amount of bound radioligand was measured on a liquid scintillation counter. The Kd and Bmax are determined by curve fitting using KaleidaGraph software (Synergy Software).

**Ligand binding studies A2A receptor:** The procedures for studying binding at recombinant A2A receptors have been described (Weiss et el., 2002, see above). Briefly, 10 µg of total membrane protein was incubated with different concentrations (0.01- 8 nM) of the A2AR antagonist 3[H]ZM241385 in 500 µl binding buffer (20 mM HEPES pH 7.4,100 mM NaCl). Adenosine deaminase (0.1 U) was added to degrade the adenosine released from the membranes and the membranes were incubated at 22°C for 1 h. Non-specific binding was determined in the presence of 10 mM theophylline. Measurements were performed in duplicate. After incubation, bound and free ligand were separated on Whatmann GF/B filters pretreated with 0.1% polyethylenimine using a Brandel cell harvester. The filters were washed three times with binding buffer and the amount of bound radioligand was measured on a liquid scintillation counter. The Kd and Bmax are determined by curve fitting using KaleidaGraph software (Synergy Software).

**Electron microscopy:** Samples were prepared for EM according to Baharaeen et al (Mycopathologia, 1982, 77(1): 19-22). For experiments wherein A2A receptor is expressed, EM pictures were taken at the end of induction of the A2A receptor. Briefly, electron microscopy was performed on an overnight grown yeast culture in YPD. Yeast cells were fixed for 2 hours on ice in 1.5% paraformaldehyde and 3% glutaraldehyde in 0.05 M sodiumCacodylate buffer, pH 7.2. After washing 3 times for 20 min in buffer, cells were treated with a 6% aqueous solution of potassium permanganate for 1 hour at room temperature. After washing 3 times for 20 min in buffer, cells were dehydrated through a graded ethanol series, including a bulk staining with 2% uranyl acetate at the 50% ethanol step, followed by embedding in Spurr's resin . Ultrathin sections of a gold interference colour were cut using an ultra microtome (ultracut E / Reichert-Jung), followed by a post-staining with uranyl acetate and lead citrate in a Leica ultrastainer and collected on formvar-coated copper slot grids. They were viewed with a transmission electron microscope 1010 (JEOL, Tokyo, Japan).

### References

André N, Cherouati N, Prual C, Steffan T, Zeder-Lutz G, Magnin T, Pattus F, Michel H, Wagner R, Reinhart C. Enhancing functional production of G protein-coupled receptors in Pichia pastoris to levels required for structural studies via a single expression screen. Protein Sci. 2006; 15(5):1115-26.
Balliet JW, Bates P. Efficient infection mediated by viral receptors incorporated into retroviral particles. J Virol. 1998; 72(1):671-6.
Boisramé A, Beckerich JM, Gaillardin C. Sls1p, an endoplasmic reticulum component, is involved in the protein translocation process in the yeast Yarrowia lipolytica. J Biol Chem. 1996; 271(20):11668-75.
Bräutigam S, Snezhkov E, Bishop DH. Formation of poliovirus-like particles by recombinant baculoviruses expressing the individual VP0, VP3, and VP1 proteins by comparison to particles derived from the expressed poliovirus polyprotein. Virology. 1993; 192(2):512-24.
Carman GM, Han GS. Roles of phosphatidate phosphatase enzymes in lipid metabolism. Trends Biochem Sci. 2006; 31(12):694-9.
Carman GM, Han GS. Phosphatidic acid phosphatase, a key enzyme in the regulation of lipid synthesis. J Biol Chem. 2009; 284(5):2593-7.
Cherezov V, Rosenbaum DM, Hanson MA, Rasmussen SG, Thian FS, Kobilka TS, Choi HJ, Kuhn P, Weis WI, Kobilka BK, Stevens RC. High-resolution crystal structure of an engineered human beta2-adrenergic G protein-coupled receptor. Science 2007; 318:1258-1265.
Chien EY, Liu W, Zhao Q, Katritch V, Han GW, Hanson MA, Shi L, Newman AH, Javitch JA, Cherezov V, Stevens RC. Structure of the human dopamine D3 receptor in complex with a D2/D3 selective antagonist. Science 2010; 330(6007):1091-5.
Connerth M, Czabany T, Wagner A, Zellnig G, Leitner E, Steyrer E, Daum G. Oleate inhibits steryl ester synthesis and causes liposensitivity in yeast. J Biol Chem. 2010; 285(35):26832-41.
De Pourcq K, De Schutter K, Callewaert N. Engineering of glycosylation in yeast and other fungi: current state and perspectives. Appl Microbiol Biotechnol. 2010; 87(5):1617-31.
Delchambre M, Gheysen D, Thines D. et al. The GAG precursor of simian immunodeficiency virus assembles into virus-like particles. EMBO J 1989; 8:2653-60.
Endres MJ, Jaffer S, Haggarty B, Turner JD, Doranz BJ, O'Brien PJ, Kolson DL, Hoxie JA. Targeting of HIV- and SIV-infected cells by CD4-chemokine receptor pseudotypes. Science. 1997; 278(5342):1462-4.
Falcón V, Garcia C, de la Rosa MC, Menéndez I, Seoane J, Grillo JM. Ultrastructural and immunocytochemical evidences of core-particle formation in the methylotrophic Pichia pastoris yeast when expressing HCV structural proteins (core-E1). Tissue Cell. 1999; 31(2):117-25.
Fickers P, Le Dall MT, Gaillardin C, Thonart P, Nicaud JM: New disruption cassettes for rapid gene disruption and marker rescue in the yeast Yarrowia lipolytica. J Microbiol Methods 2003, 55(3):727-737.
French TJ, Marshall JJ, Roy P. Assembly of double-shelled, virus-like particles of bluetongue virus by the simultaneous expression of four structural proteins. J Virol 1990; 64:5695-700.
Gheysen D, Jacobs E, de Foresta F et al. Assembly and release of HIV-1 precursor Pr55gag virus-like particles from recombinant baculovirus-infected insect cells. Cell 1989; 59:103-12.
Grimsey N, Han GS, O'Hara L, Rochford JJ, Carman GM, Siniossoglou S. Temporal and spatial regulation of the phosphatidate phosphatases lipin 1 and 2. J Biol Chem. 2008; 283(43):29166-74.
Grisshammer R, Duckworth R, Henderson R. Expression of a rat neurotensin receptor in Escherichia coli. Biochem J. 1993; 295 (Pt 2):571-6.
Guerfal M, Ryckaert S, Jacobs PP, Ameloot P, Van Craenenbroeck K, Derycke R, Callewaert N: The HAC1 gene from Pichia pastoris: characterization and effect of its overexpression on the production of secreted, surface displayed and membrane proteins. Microb Cell Fact. 2010, 9:49.
Gurramkonda C, Adnan A, Gäbel T, Lünsdorf H, Ross A, Nemani SK, Swaminathan S, Khanna N, Rinas U. Simple high-cell density fed-batch technique for high-level recombinant protein production with Pichia pastoris: Application to intracellular production of Hepatitis B surface antigen. Microb Cell Fact. 2009; 8:13.
Hadiji-Abbes N, Borchani-Chabchoub I, Triki H, Ellouz R, Gargouri A, Mokdad-Gargouri R. Expression of HBsAg and preS2-S protein in different yeast based system: a comparative analysis. Protein Expr Purif. 2009; 66(2):131-7.
Han GS, Wu WI, Carman GM. The Saccharomyces cerevisiae Lipin homolog is a Mg2+-dependent phosphatidate phosphatase enzyme. J Biol Chem. 2006; 281(14):9210-8.
Han GS, Siniossoglou S, Carman GM. The cellular functions of the yeast lipin homolog PAH1p are dependent on its phosphatidate phosphatase activity. J Biol Chem. 2007; 282(51):37026-35.
Han GS, O'Hara L, Carman GM, Siniossoglou S. An unconventional diacylglycerol kinase that regulates phospholipid synthesis and nuclear membrane growth. J Biol Chem. 2008a; 283(29):20433-42.
Han GS, O'Hara L, Siniossoglou S, Carman GM. Characterization of the yeast DGK1-encoded CTP-dependent diacylglycerol kinase. J Biol Chem. 2008b; 283(29):20443-53.
Hellwig S, Drossard J, Twyman RM, Fischer R. Plant cell cultures for the production of recombinant proteins. Nat Biotechnol. 2004; 22(11):1415-22.
Huang T-K, McDonald KA. Bioreactor engineering for recombinant protein production in plant cell suspension cultures. Biochemical Engineering Journal 2009; 45(3):168-184.
Insel PA, Tang CM, Hahntow I, Michel MC. Impact of GPCRs in clinical medicine: genetic variants and drug targets. Biochim Biophys Acta. 2007; 1768(4): 994-1005.
Jaakola VP, Griffith MT, Hanson MA, Cherezov V, Chien EY, Lane JR, Ijzerman AP, Stevens RC. The 2.6 angstrom crystal structure of a human A2A adenosine receptor bound to an antagonist. Science 2008; 322:1211-1217.
Jacobs PP, Callewaert N. N-glycosylation engineering of biopharmaceutical expression systems. Curr Mol Med. 2009; 9(7):774-800.
Jose AM, Koelle MR. Domains, amino acid residues, and new isoforms of Caenorhabditis elegans diacylglycerol kinase 1 (DGK-1) important for terminating diacylglycerol signaling in vivo. J Biol Chem. 2005; 280(4):2730-6.
Katagiri T, Mizoguchi T, Shinozaki K. Molecular cloning of a cDNA encoding diacylglycerol kinase (DGK) in Arabidopsis thaliana. Plant Mol Biol. 1996; 30(3):647-53.
Kibenge FS, Qian B, Nagy E, Cleghorn JR, Wadowska D. Formation of virus-like particles when the polyprotein gene (segment A) of infectious bursal disease virus is expressed in insect cells. Can J Vet Res. 1999; 63(1):49-55.
Liu W, Jiang H, Zhou J, Yang X, Tang Y, Fang D, Jiang L. Recombinant dengue virus-like particles from Pichia pastoris: efficient production and immunological properties. Virus Genes. 2010; 40(1):53-9.
Lundstrom K, Wagner R, Reinhart C, Desmyter A, Cherouati N, Magnin T, Zeder-Lutz G, Courtot M, Prual C, André N, Hassaine G, Michel H, Cambillau C, Pattus F. Structural genomics on membrane proteins: comparison of more than 100 GPCRs in 3 expression systems. J Struct Funct Genomics. 2006; 7(2):77-91.
Madzak C, Gaillardin C, Beckerich JM. Heterologous protein expression and secretion in the nonconventional yeast Yarrowia lipolytica: a review. J Biotechnol 2004, 109(1-2):63-81.
Miyanohara A, Imamura T, Araki M. et al. Expression of hepatitis B virus core antigen gene in Saccharomyces cerevisiae: synthesis of two polypeptides translated from different initiation codons. J Virol 1986; 59:176-80.
Noad R, Roy P. Virus-like particles as immunogens. Trends Microbiol. 2003; 11(9):438-44.
O'Keefe DO, Paiva AM. Assay for recombinant hepatitis B surface antigen using reversed-phase high-performance liquid chromatography. Anal Biochem. 1995; 230(1):48-54.
Palczewski K, Kumasaka T, Hori T, Behnke CA, Motoshima H, Fox BA, Le Trong I, Teller DC, Okada T, Stenkamp RE, Yamamoto M, Miyano M. Crystal structure of rhodopsin: A G protein-coupled receptor. Science. 2000; 289(5480):739-45.
Penel S, Arigon AM, Dufayard JF, Sertier AS, Daubin V, Duret L, Gouy M, Perrière G. Databases of homologous gene families for comparative genomics. BMC Bioinformatics. 2009; 10 Suppl 6:S3.
Péterfy M, Phan J, Xu P, Reue K. Lipodystrophy in the fld mouse results from mutation of a new gene encoding a nuclear protein, lipin. Nat Genet. 2001; 27(1):121-4.
Rasmussen SG, Choi HJ, Rosenbaum DM, Kobilka TS, Thian FS, Edwards PC, Burghammer M, Ratnala VR, Sanishvili R, Fischetti RF, Schertler GF, Weis WI, Kobilka BK. Crystal structure of the human beta2 adrenergic G-protein-coupled receptor. Nature 2007; 450:383-387.
Reeves PJ, Thurmond RL, Khorana HG. Structure and function in rhodopsin: high level expression of a synthetic bovine opsin gene and its mutants in stable mammalian cell lines. Proc Natl Acad Sci U S A. 1996; 93(21):11487-92.
Reeves PJ, Callewaert N, Contreras R, Khorana HG. Structure and function in rhodopsin: high-level expression of rhodopsin with restricted and homogeneous N-glycosylation by a tetracycline-inducible N-acetylglucosaminyltransferase I-negative HEK293S stable mammalian cell line. Proc Natl Acad Sci U S A. 2002; 99(21):13419-24.
Roy P, Noad R. Virus-like particles as a vaccine delivery system: myths and facts. Hum Vaccin. 2008; 4(1):5-12.
Sakane F, Imai S, Kai M, Yasuda S, Kanoh H. Diacylglycerol kinases: why so many of them? Biochim Biophys Acta. 2007; 1771(7):793-806.
Sakuragi S, Goto T, Sano K, Morikawa Y. HIV type 1 Gag virus-like particle budding from spheroplasts of Saccharomyces cerevisiae. Proc Natl Acad Sci U S A. 2002; 99(12):7956-61.
Salom D, Le Trong I, Pohl E, Ballesteros JA, Stenkamp RE, Palczewski K, Lodowski DT. Improvements in G protein-coupled receptor purification yield light stable rhodopsin crystals. J Struct Biol. 2006; 156(3):497-504.
Santi L, Huang Z, Mason H. Virus-like particles production in green plants. Methods. 2006; 40(1):66-76.
Santos-Rosa H, Leung J, Grimsey N, Peak-Chew S, Siniossoglou S. The yeast lipin Smp2 couples phospholipid biosynthesis to nuclear membrane growth. EMBO J. 2005; 24(11):1931-41.
Siniossoglou S, Santos-Rosa H, Rappsilber J, Mann M, Hurt E. A novel complex of membrane proteins required for formation of a spherical nucleus. EMBO J. 1998; 17(22):6449-64.
Smith AJ, Srinivasakumar N, Hammarskjöld ML, Rekosh D. Requirements for incorporation of Pr160gag- pol from human immunodeficiency virus type 1 into virus-like particles. J Virol. 1993; 67(4):2266-75.
Sugrue RJ, Fu J, Howe J, Chan YC. Expression of the dengue virus structural proteins in Pichia pastoris leads to the generation of virus-like particles. J Gen Virol. 1997; 78 (Pt 8):1861-6.
Tritel M, Resh MD. Kinetic analysis of human immunodeficiency virus type 1 assembly reveals the presence of sequential intermediates. J Virol. 2000; 74(13):5845-55.
Vaultier MN, Cantrel C, Guerbette F, Boutté Y, Vergnolle C, Ciçek D, Bolte S, Zachowski A, Ruelland E. The hydrophobic segment of Arabidopsis thaliana cluster I diacylglycerol kinases is sufficient to target the proteins to cell membranes. FEBS Lett. 2008; 582(12):1743-8.
Warne T, Serrano-Vega MJ, Baker JG, Moukhametzianov R, Edwards PC, Henderson R, Leslie AG, Tate CG, Schertler GF. Structure of a beta1-adrenergic G-protein-coupled receptor. Nature 2008; 454:486-491.
Wildt S, Gerngross TU. The humanization of N-glycosylation pathways in yeast. Nat Rev Microbiol. 2005; 3(2):119-28.
Willis S, Davidoff C, Schilling J, Wanless A, Doranz BJ, Rucker J. Virus-like particles as quantitative probes of membrane protein interactions. Biochemistry. 2008; 47(27):6988-90.
Wu B, Chien EY, Mol CD, Fenalti G, Liu W, Katritch V, Abagyan R, Brooun A, Wells P, Bi FC, Hamel DJ, Kuhn P, Handel TM, Cherezov V, Stevens RC. Structures of the CXCR4 chemokine GPCR with small-molecule and cyclic peptide antagonists. Science. 2010; 330(6007):1066-71.

## Claims

1. A method of producing a protein in a eukaryotic cell, comprising the steps of: providing a eukaryotic cell
- wherein expression and/or activity of an endogenous phosphatidic acid phosphatase is inhibited; and/or
- overexpressing a diacylglycerol kinase
to the extent that the endoplasmic reticulum is expanded in the cell, the cell further comprising an endogenous nucleic acid sequence under control of an exogenous promoter, said nucleic acid encoding a protein, or the cell further comprising an exogenous nucleic acid sequence encoding a receptor or secreted protein, in conditions suitable for expressing the protein.

2. The method according to claim 1, wherein the endogenous phosphatidic acid phosphatase is PAH1 or a homolog thereof.

3. The method according to any one of claims 1 to 2, wherein the expression and/or activity of the endogenous phosphatidic acid phosphatase is inhibited through disruption of the endogenous phosphatidic acid phosphatase gene at nucleic acid level.

4. The method according to any one of claims 1 to 2, wherein the expression and/or activity of the endogenous phosphatidic acid phosphatase is inhibited through an inhibitory RNA directed to the endogenous phosphatidic acid phosphatase gene transcript.

5. The method according to any one of claims 1 to 4, wherein the diacylglycerol kinase is DGK1 or a homolog thereof.

6. The method according to any one of claims 1 to 5, wherein endogenous diacylglycerol kinase is overexpressed.

7. The method according to any one of claims 1 to 5, wherein an exogenous diacylglycerol kinase is overexpressed.

8. The method according to any one of claims 1 to 7, wherein the overexpression is inducible.

9. The method according to any one of claims 1 to 8, wherein the protein is a membrane-bound protein.

10. The method of any one of claims 1 to 9, wherein the protein is a receptor, in particular a GPCR.

11. The method of any one of claims 1 to 10, wherein the eukaryotic cell is a yeast cell, in particular a Yarrowia or Pichia cell, and the protein is to be expressed and isolated from the cell.

12. The method according to claim 11, wherein the yeast cell is a glyco-engineered yeast cell.

13. The method of any one of claims 1 to 10, wherein the eukaryotic cell is a mammalian cell, in particular a Hek293S cell.

14. The method according to any one of claims 1 to 13, further comprising the step of isolating the expressed protein.

15. A method of producing a virus-like particle in a eukaryotic cell, comprising the steps of: providing a eukaryotic cell
- wherein expression and/or activity of an endogenous phosphatidic acid phosphatase is inhibited; and/or
- overexpressing a diacylglycerol kinase,
to the extent that the endoplasmic reticulum is expanded in the cell, the cell comprising one or more nucleic acid sequences encoding the one or more proteins making up the virus-like particle, in conditions suitable for expressing the one or more proteins.

16. The method according to claim 15, wherein the virus-like particle further encompasses lipids, such as in a lipoparticle.

17. The method according to claim 15 or 16, wherein the virus-like particles are used for production of vaccines or for production of membrane proteins.

18. The method according to any one of claims 15 to 17, wherein the virus-like particles are based on a hepatitis virus, particularly HCV virus, on an encephalitis virus, particularly Japanese encephalitis virus, or on a Dengue virus.

19. A eukaryotic cell deficient in expression and/or activity of an endogenous phosphatidic acid phosphatase, and/or overexpressing an endogenous diacylglycerol kinase by means of an exogenous promoter and/or genetically engineered so as to express a diacylglycerol kinase that said cell does not normally express, to the extent that the endoplasmic reticulum is expanded in the cell, the cell further comprising an endogenous nucleic acid sequence under control of an exogenous promoter or further comprising an exogenous nucleic acid sequence encoding a receptor or secreted protein to be expressed and isolated from the cell.

20. The eukaryotic cell according to claim 19, wherein the cell is a yeast cell, in particular a Yarrowia or Pichia cell.

21. The eukaryotic cell according to claims 19 or 20, wherein the endogenous phosphatidic acid phosphatase is PAH1 or a homolog thereof and/or wherein the diacylglycerol kinase is DGK1 or a homolog thereof.

22. A cell culture of cells according to any one of claims 19 to 21.

## Patentansprüche

1. Verfahren zur Produktion eines Proteins in einer eukaryotischen Zelle, das die folgenden Schritte umfasst: Bereitstellen einer eukaryotischen Zelle,
- wobei eine Expression und/oder eine Aktivität einer endogenen Phosphatidsäurephosphatase gehemmt wird; und/oder
- Überexprimieren einer Diacylglycerinkinase,
in dem Ausmaß, dass das endoplasmatische Retikulum in der Zelle expandiert wird, wobei die Zelle weiterhin eine endogene Nukleinsäuresequenz unter der Kontrolle eines exogenen Promotors umfasst, wobei die Nukleinsäure ein Protein kodiert, oder die Zelle weiterhin eine exogene Nukleinsäuresequenz umfasst, die einen Rezeptor oder ein sezerniertes Protein kodiert, unter Bedingungen, die zum Exprimieren des Proteins geeignet sind.

2. Verfahren nach Anspruch 1, wobei die endogene Phosphatidsäurephosphatase PAH1 oder ein Homolog davon ist.

3. Verfahren nach einem der Ansprüche 1 bis 2, wobei die Expression und/oder die Aktivität der endogenen Phosphatidsäurephosphatase durch Disruption des Gens der endogenen Phosphatidsäurephosphatase auf der Nukleinsäure-Ebene gehemmt werden.

4. Verfahren nach einem der Ansprüche 1 bis 2, wobei die Expression und/oder die Aktivität der endogenen Phosphatidsäurephosphatase durch eine hemmende RNA, die auf das Transkript des Gens der endogenen Phosphatidsäurephosphatase ausgerichtet ist, gehemmt werden.

5. Verfahren nach einem der Ansprüche 1 bis 4, wobei die Diacylglycerinkinase DGK1 oder ein Homolog davon ist.

6. Verfahren nach einem der Ansprüche 1 bis 5, wobei endogene Diacylglycerinkinase überexprimiert wird.

7. Verfahren nach einem der Ansprüche 1 bis 5, wobei eine exogene Diacylglycerinkinase überexprimiert wird.

8. Verfahren nach einem der Ansprüche 1 bis 7, wobei die Überexpression induzierbar ist.

9. Verfahren nach einem der Ansprüche 1 bis 8, wobei das Protein ein membrangebundenes Protein ist.

10. Verfahren nach einem der Ansprüche 1 bis 9, wobei das Protein ein Rezeptor, insbesondere ein GPCR ist.

11. Verfahren nach einem der Ansprüche 1 bis 10, wobei die eukaryotische Zelle eine Hefezelle, insbesondere eine Yarrowia- oder Pichia-Zelle ist und das Protein aus der Zelle exprimiert und isoliert werden soll.

12. Verfahren nach Anspruch 11, wobei die Hefezelle eine Glyko-engineerte Hefezelle ist.

13. Verfahren nach einem der Ansprüche 1 bis 10, wobei die eukaryotische Zelle eine Säugerzelle, insbesondere eine Hek293S-Zelle ist.

14. Verfahren nach einem der Ansprüche 1 bis 13, das weiterhin den Schritt eines Isolierens des exprimierten Proteins umfasst.

15. Verfahren zur Produktion eines virusähnlichen Partikels in einer eukaryotischen Zelle, das die folgenden Schritte umfasst: Bereitstellen einer eukaryotischen Zelle,
- wobei eine Expression und/oder eine Aktivität einer endogenen Phosphatidsäurephosphatase gehemmt wird; und/oder
- Überexprimieren einer Diacylglycerinkinase,
in dem Ausmaß, dass das endoplasmatische Retikulum in der Zelle expandiert wird, wobei die Zelle eine oder mehrere Nukleinsäuresequenzen umfasst, die das eine oder die mehreren Proteine, die das virusähnliche Partikel bilden, exprimieren, unter Bedingungen, die zum Exprimieren des einen oder der mehreren Proteine geeignet sind.

16. Verfahren nach Anspruch 15, wobei das virusähnliche Partikel weiterhin Lipide, wie in einem Lipopartikel, umspannt.

17. Verfahren nach Anspruch 15 oder 16, wobei die virusähnlichen Partikel zur Produktion von Impfstoffen oder zur Produktion von Membranproteinen verwendet werden.

18. Verfahren nach einem der Ansprüche 15 bis 17, wobei die virusähnlichen Partikel auf einem Hepatitisvirus, insbesondere HCV-Virus, auf einem Enzephalitisvirus, insbesondere japanischem Enzephalitisvirus, oder auf einem Dengue-Virus basieren.

19. Eukaryotische Zelle, der es an Expression und/oder Aktivität einer endogenen Phosphatidsäurephosphatase mangelt und/oder die eine endogene Diacylglycerinkinase mittels eines exogenen Promotors überexprimiert und/oder gentechnisch verändert ist, um eine Diacylglycerinkinase zu exprimieren, die die Zelle normalerweise nicht exprimiert, in dem Ausmaß, dass das endoplasmatische Retikulum in der Zelle expandiert wird, wobei die Zelle weiterhin eine endogene Nukleinsäuresequenz unter der Kontrolle eines exogenen Promotors umfasst oder weiterhin eine exogene Nukleinsäuresequenz umfasst, die einen Rezeptor oder ein sezerniertes Protein kodiert, das aus der Zelle exprimiert und isoliert werden soll.

20. Eukaryotische Zelle nach Anspruch 19, wobei die Zelle eine Hefezelle, insbesondere eine Yarrowia- oder Pichia-Zelle ist.

21. Eukaryotische Zelle nach Anspruch 19 oder 20, wobei die endogene Phosphatidsäurephosphatase PAH1 oder ein Homolog davon ist und/oder wobei die Diacylglycerinkinase DGK1 oder ein Homolog davon ist.

22. Zellkultur von Zellen nach einem der Ansprüche 19 bis 21.

## Revendications

1. Procédé de production d'une protéine dans une cellule eucaryote, comprenant les étapes consistant à : fournir une cellule eucaryote
- dans lequel l'expression et/ou l'activité d'une acide phosphatidique phosphatase endogène est inhibée ; et/ou
- surexprimer une diacylglycérol kinase
dans la mesure où le réticulum endoplasmique est dilaté dans la cellule, la cellule comprenant en outre une séquence d'acide nucléique endogène sous le contrôle d'un promoteur exogène, ledit acide nucléique codant pour une protéine, ou la cellule comprenant en outre une séquence d'acide nucléique exogène codant pour un récepteur ou une protéine secrétée, dans des conditions convenant à l'expression de la protéine.

2. Procédé selon la revendication 1, dans lequel l'acide phosphatidique phosphatase endogène est PAH1 ou un homologue de celle-ci.

3. Procédé selon l'une quelconque des revendications 1 à 2, dans lequel l'expression et/ou l'activité de l'acide phosphatidique phosphatase endogène est inhibée par la perturbation du gène de l'acide phosphatidique phosphatase endogène au niveau de l'acide nucléique.

4. Procédé selon l'une quelconque des revendications 1 à 2, dans lequel l'expression et/ou l'activité de l'acide phosphatidique phosphatase endogène est inhibée par un ARN inhibiteur dirigé vers le produit de la transcription du gène de l'acide phosphatidique phosphatase endogène.

5. Procédé selon l'une quelconque des revendications 1 à 4, dans lequel la diacylglycérol kinase est DGK1 ou un homologue de celle-ci.

6. Procédé selon l'une quelconque des revendications 1 à 5, dans lequel la diacylglycérol kinase endogène est surexprimée.

7. Procédé selon l'une quelconque des revendications 1 à 5, dans lequel une diacylglycérol kinase exogène est surexprimée.

8. Procédé selon l'une quelconque des revendications 1 à 7, dans lequel la surexpression est inductible.

9. Procédé selon l'une quelconque des revendications 1 à 8, dans lequel la protéine est une protéine fixée à la membrane.

10. Procédé selon l'une quelconque des revendications 1 à 9, dans lequel la protéine est un récepteur, notamment un RCPG.

11. Procédé selon l'une quelconque des revendications 1 à 10, dans lequel la cellule eucaryote est une cellule de levure, notamment une cellule Yarrowia ou Pichia, et la protéine doit être exprimée et isolée de la cellule.

12. Procédé selon la revendication 11, dans lequel la cellule de levure est une cellule de levure conçue par glyco-ingéniérie.

13. Procédé selon l'une quelconque des revendications 1 à 10, dans lequel la cellule eucaryote est une cellule mammifère, notamment une cellule Hek293S.

14. Procédé selon l'une quelconque des revendications 1 à 13, comprenant en outre l'étape consistant à isoler la protéine exprimée.

15. Procédé de production d'une particule de type virus dans une cellule eucaryote, comprenant les étapes consistant à : fournir une cellule eucaryote
- dans lequel l'expression et/ou l'activité d'une acide phosphatidique phosphatase endogène est inhibée ; et/ou
- surexprimer une diacylglycérol kinase
dans la mesure où le réticulum endoplasmique est dilaté dans la cellule, la cellule comprenant une ou plusieurs séquences d'acide nucléique codant pour la ou les protéines constituant la particule de type virus, dans des conditions convenant à l'expression de la ou des protéines.

16. Procédé selon la revendication 15, dans lequel la particule de type virus englobe en outre des lipides tels que dans une lipoparticule.

17. Procédé selon la revendication 15 ou 16, dans lequel les particules de type virus sont utilisées pour la production de vaccins ou pour la production de protéines membranaires.

18. Procédé selon l'une quelconque des revendications 15 à 17, dans lequel les particules de type virus sont basées sur un virus d'hépatite, notamment le virus HCV, sur un virus d'encéphalite, notamment le virus de l'encéphalite japonaise, ou sur un virus de la dengue.

19. Cellule eucaryote à l'expression et/ou l'activité insuffisante d'une acide phosphatidique phosphatase endogène et/ou surexprimant une diacylglycérol kinase endogène au moyen d'un promoteur exogène et/ou créée par génie génétique de manière à exprimer une diacylglycérol kinase que ladite cellule n'exprime normalement pas, dans la mesure où le réticulum endoplasmique est dilaté dans la cellule, la cellule comprenant en outre une séquence d'acide nucléique endogène sous le contrôle d'un promoteur exogène ou comprenant en outre une séquence d'acide nucléique exogène codant pour un récepteur ou une protéine secrétée devant être exprimée et isolée de la cellule.

20. Cellule eucaryote selon la revendication 19, dans laquelle la cellule est une cellule de levure, notamment une cellule Yarrowia ou Pichia.

21. Cellule eucaryote selon la revendication 19 ou 20, dans laquelle l'acide phosphatidique phosphatase endogène est PAH1 ou un homologue de celle-ci et/ou dans laquelle la diacylglycérol kinase est DGK1 ou un homologue de celle-ci.

22. Culture cellulaire de cellules selon l'une quelconque des revendications 19 à 21.


### SEQUENCE LISTING

<110> VIB VZW UNIVERSITEIT GENT
<120> Increased protein expression through increased membrane formation
<130> NCA/PAH1/346
<150> EP 10166271.6
   <151> 2010-06-17
<160> 21
<170> PatentIn version 3.5
<210> 1
   <211> 3772
   <212> DNA
   <213> Yarrowia lipolytica
<400> 1
   aggctgtatt tctgtgtgct agtcggcact tttcatccac tccccacgtg atcacatcga      60
   ttggaacagc aaacccctcc ttgtacttgc tgcatatctg tatgtatacc gaactctgtg     120
   aaggtgccca acagatcatc accacacaac cacacgatgc agtacgttgg gcgagccatt     180
   ggaaccgttt ccaagacctg gaactccatc aaccctgcaa cactcagtgg tgcaattgac     240
   atcattgtgg tcgagcggga agacggtgag tatggccatc aagaggctgt tggagaagtg     300
   gagctgatgc agtgaccgac ggtgacttac aggatgcaag ttgagagggt gatagagaag     360
   tggactttga gaacagagag tgggtgcaag tggccttaca gttgttactt ctaactggca     420
   gttacaccac tcttgtaact atcatggtcc gtatgatgtg accacatgtt gtacaccatg     480
   actgtaatcg tgcccaggtg gcaacagtat gattcacagt tgttatgtca tatggacaaa     540
   gttgtaagag gccaatacaa ctggagcaac cagcttgttt cgggtttcct gtcaactact     600
   tcccactcac tcgaaacact caacacatga acacaacgtg gtggcgtaac acctcatccc     660
   cataaccacc atatccacac actaacccag gtgaactaaa ctgctctcct ttccatgtgc     720
   ggtttggaat cttttccctg ctgcgaccgt cccagaaaaa ggtcgagttc tgtgtcaatg     780
   gcaccaagac cgagctgccc atgaaggtgg gcgatggagg agaagtcttc tttgtgttcg     840
   agaccgacgc agacgtgccc gaagagctcc tgacatcccc cgtcatttct ccctcttcgt     900
   cgccatcctg gggccaggag gaaggcgggg atggtgagcc ggactacctt gctctgaacg     960
   actctaaaca gggtggcgac agcaagcacg gcagatcgcc ctcggagggc ccaccattca    1020
   gatcaccttc ggcggatcac ttacatgaga tgggcagctt cgatgatgag aatgaccctg    1080
   aggtgaacag aagacaacgt gcgagcacgg cagctccaga gcctgttcct ggttcgttga    1140
   aacacccagc cactatctcg gaaggcatct cttcggcctc gttttccaac agcgatactg    1200
   atcgaacaga cacttctgga cccacagaga cagaacccac agagctcaca gagcctacag    1260
   agcccacaga gcccacagag cctctggatc ttgagcagag tctccaccgg gctgccactt    1320
   ctcccgcccc ttcgtccgag gagatttggg agaaggcccg tgcactgtcc aagaaactca 1380
   catcagaaaa cattcagagt aaaatctccg acaacggaga cattattctg gatatgactg 1440
   gttacaagta cgaccacgag gacgtgagtc gatcagagga gctggtcaag aaaatcctcg 1500
   ctgaggaact gggagaagac agagacctgt cccacatcct ggttgaagac gaggagggta 1560
   accttgtgat tcagagcgct ggagacagcc accatcacga gcatatgagc tcgcccgagt 1620
   ctctggccca ctcccctcag cccctccctt cttctaacct tccgtctcag gcctcggaca 1680
   acaagcacta cgccaagacc atccgtttga cgtctgacca gctcaagtct ctggatctca 1740
   agcccggcaa gaacgaggtc acctttgctg tcaataacgg caagacgtcg tgttcggccc 1800
   agctgttcta ctggaagtac gacattcctg ttgtcatttc cgacattgat ggcacgatca 1860
   ccaagtccga tgctctgggc catctgctca ccatgatggg ccgagactgg acccacaccg 1920
   gcgtggccaa gctcttttcc gatatcagag ccaacgggta taatatcatg tatctgacag 1980
   cacgatcagt gggacaggca gatgcaacca gggcatatct aggcggtgtt gaccagtttg 2040
   gcttcaagct gcctccagga cccgtcatct tgtcgcctga tagaaccctg gcggctctca 2100
   agagagaggt gattcttaag aaacctgagg tattcaagat ggcgtgtctg cgggacatta 2160
   agtcgctgtt tggcgagacc gaagacgcca ccaatccatt ctacgctgga tttggcaacc 2220
   gaatcaccga cgcgttgtcg tatagatctg tcggtgtgcc gtcgtctaga atcttcacaa 2280
   tcaactcgaa cgccgaggtc catatggagc tgcttgaact ggctggctac aagtcctcgt 2340
   atgtccacat tgccgatctt gtcgaccact ttttccctcc ggaaagcgag ttcacgacca 2400
   ttcaggagga aaaatacacg gacgtcaact actggcgaga tcccattatt gacctgtctg 2460
   atctgaccga cgacgagctg actgacgatg atgagctctc caagtcgccc aagtcgccca 2520
   gatctcctag aagcccgcgg gccggttcgg caggctccag cgcggctccc tcaggctcgg 2580
   gcgccgaccc tgccggaccc tccgagccga aggactccgc gaacccgtcg aagttcagct 2640
   ataagaaggc tcctacgaac tctcgattcc agcccgtttc gtacgatctt gatcttgacg 2700
   acggatacga gtacgacgat gacgatgact atgatgacga tgaggagttt gtggacgctg 2760
   agagcgacgc gctggaggag gatgacgacg atgatgatga cgtcgaccta gacaacgact 2820
   ctgaccactc ccctgtcaag ccgccctcgc agatgcagcg agtcatcaac aagactattg 2880
   aggacaacaa gggcctgcac atggatgagg atgacgttca aaaagccatg aaggccctga 2940
   agatggaacg agcaagcatc aatcctgagt aaataggaaa tcaagcataa atatatccgt 3000
   actgtacacc gtagtttgtg acgtcgaagg ggagggggac aagagagtga aagtgtagct 3060
   acttgtagta tgtataagtt gtgcgtacct gaatgtcacg ggacctgcga tacgaatgct 3120
   ggtgagaccc aaccatggtt cccgctagaa agccaagata ttatctggat ctacagctga 3180
   gtttgacgtt attttttttt ccctcctgaa aggctttgtt aggtttgcat cgttcatgta 3240
   tttggtgtac acctttttcg ggttccgatg gtgcttccat aggtggagga gcaggtcaga      3300
   gacacattgt ttgtggatag cggattgtta tgccccgagt ggagcatcat tttaatcgca      3360
   ggagtttatt atatactgtg tatactgtgc actaattttt cccctgtctc ctctacactc      3420
   ccttacgtca ttaaatggcc gctactgtat tcacactctt ccgacccgga tcagataaat      3480
   ttttcaagaa cttttttatc tgatttgtat gcatggtttg ggggcaagta agccgtacat      3540
   aggaggcctg gaagtagggg caaaagtgga gagtccctgt aaccactggt ttgtgcaatt      3600
   ttgggggtcg caaatacatt gccacaacgt gggcgccagg agtttacaga cgagtctata      3660
   tacgggcggt cttagatgaa ttttactact gcacgtgtga gcaatttgag ggcctgtgtt      3720
   gacgggtgcg agtttaacgt gcagggttca gtggggtgat ttgagccgac aa              3772
<210> 2
   <211> 723
   <212> PRT
   <213> Yarrowia lipolytica
<400> 2
   Met Lys Val Gly Asp Gly Gly Glu Val Phe Phe Val Phe Glu Thr Asp
   1               5                   10                  15
   Ala Asp Val Pro Glu Glu Leu Leu Thr Ser Pro Val Ile Ser Pro Ser
               20                  25                  30
   Ser Ser Pro Ser Trp Gly Gln Glu Glu Gly Gly Asp Gly Glu Pro Asp
           35                  40                  45
   Tyr Leu Ala Leu Asn Asp Ser Lys Gln Gly Gly Asp Ser Lys His Gly
       50                  55                  60
   Arg Ser Pro Ser Glu Gly Pro Pro Phe Arg Ser Pro Ser Ala Asp His
   65                  70                  75                  80
   Leu His Glu Met Gly Ser Phe Asp Asp Glu Asn Asp Pro Glu Val Asn
                   85                  90                  95
   Arg Arg Gln Arg Ala Ser Thr Ala Ala Pro Glu Pro Val Pro Gly Ser
               100                 105                 110
   Leu Lys His Pro Ala Thr Ile Ser Glu Gly Ile Ser Ser Ala Ser Phe
           115                 120                 125
   Ser Asn Ser Asp Thr Asp Arg Thr Asp Thr Ser Gly Pro Thr Glu Thr
       130                 135                 140
   Glu Pro Thr Glu Leu Thr Glu Pro Thr Glu Pro Thr Glu Pro Thr Glu
   145                 150                 155                 160
   Pro Leu Asp Leu Glu Gln Ser Leu His Arg Ala Ala Thr Ser Pro Ala
   165 170 175
   Pro Ser Ser Glu Glu Ile Trp Glu Lys Ala Arg Ala Leu Ser Lys Lys
   180 185 190
   Leu Thr Ser Glu Asn Ile Gln Ser Lys Ile Ser Asp Asn Gly Asp Ile
   195 200 205
   Ile Leu Asp Met Thr Gly Tyr Lys Tyr Asp His Glu Asp Val Ser Arg
   210 215 220
   Ser Glu Glu Leu Val Lys Lys Ile Leu Ala Glu Glu Leu Gly Glu Asp
   225 230 235 240
   Arg Asp Leu Ser His Ile Leu Val Glu Asp Glu Glu Gly Asn Leu Val
   245 250 255
   Ile Gln Ser Ala Gly Asp Ser His His His Glu His Met Ser Ser Pro
   260 265 270
   Glu Ser Leu Ala His Ser Pro Gln Pro Leu Pro Ser Ser Asn Leu Pro
   275 280 285
   Ser Gln Ala Ser Asp Asn Lys His Tyr Ala Lys Thr Ile Arg Leu Thr
   290 295 300
   Ser Asp Gln Leu Lys Ser Leu Asp Leu Lys Pro Gly Lys Asn Glu Val
   305 310 315 320
   Thr Phe Ala Val Asn Asn Gly Lys Thr Ser Cys Ser Ala Gln Leu Phe
   325 330 335
   Tyr Trp Lys Tyr Asp Ile Pro Val Val Ile Ser Asp Ile Asp Gly Thr
   340 345 350
   Ile Thr Lys Ser Asp Ala Leu Gly His Leu Leu Thr Met Met Gly Arg
   355 360 365
   Asp Trp Thr His Thr Gly Val Ala Lys Leu Phe Ser Asp Ile Arg Ala
   370 375 380
   Asn Gly Tyr Asn Ile Met Tyr Leu Thr Ala Arg Ser Val Gly Gln Ala
   385 390 395 400
   Asp Ala Thr Arg Ala Tyr Leu Gly Gly Val Asp Gln Phe Gly Phe Lys
   405 410 415
   Leu Pro Pro Gly Pro Val Ile Leu Ser Pro Asp Arg Thr Leu Ala Ala
               420                 425                 430
   Leu Lys Arg Glu Val Ile Leu Lys Lys Pro Glu Val Phe Lys Met Ala
           435                 440                 445
   Cys Leu Arg Asp Ile Lys Ser Leu Phe Gly Glu Thr Glu Asp Ala Thr
       450                 455                 460
   Asn Pro Phe Tyr Ala Gly Phe Gly Asn Arg Ile Thr Asp Ala Leu Ser
   465                 470                 475                 480
   Tyr Arg Ser Val Gly Val Pro Ser Ser Arg Ile Phe Thr Ile Asn Ser
                   485                 490                 495
   Asn Ala Glu Val His Met Glu Leu Leu Glu Leu Ala Gly Tyr Lys Ser
               500                 505                 510
   Ser Tyr Val His Ile Ala Asp Leu Val Asp His Phe Phe Pro Pro Glu
           515                 520                 525
   Ser Glu Phe Thr Thr Ile Gln Glu Glu Lys Tyr Thr Asp Val Asn Tyr
       530                 535                 540
   Trp Arg Asp Pro Ile Ile Asp Leu Ser Asp Leu Thr Asp Asp Glu Leu
   545                 550                 555                 560
   Thr Asp Asp Asp Glu Leu Ser Lys Ser Pro Lys Ser Pro Arg Ser Pro
                   565                 570                 575
   Arg Ser Pro Arg Ala Gly Ser Ala Gly Ser Ser Ala Ala Pro Ser Gly
               580                 585                 590
   Ser Gly Ala Asp Pro Ala Gly Pro Ser Glu Pro Lys Asp Ser Ala Asn
           595                 600                 605
   Pro Ser Lys Phe Ser Tyr Lys Lys Ala Pro Thr Asn Ser Arg Phe Gln
       610                 615                 620
   Pro Val Ser Tyr Asp Leu Asp Leu Asp Asp Gly Tyr Glu Tyr Asp Asp
   625                 630                 635                 640
   Asp Asp Asp Tyr Asp Asp Asp Glu Glu Phe Val Asp Ala Glu Ser Asp
                   645                 650                 655
   Ala Leu Glu Glu Asp Asp Asp Asp Asp Asp Asp Val Asp Leu Asp Asn
               660                 665                 670
   Asp Ser Asp His Ser Pro Val Lys Pro Pro Ser Gln Met Gln Arg Val
   675 680 685
   Ile Asn Lys Thr Ile Glu Asp Asn Lys Gly Leu His Met Asp Glu Asp
   690 695 700
   Asp Val Gln Lys Ala Met Lys Ala Leu Lys Met Glu Arg Ala Ser Ile
   705 710 715 720
   Asn Pro Glu
<210> 3
   <211> 4325
   <212> DNA
   <213> Pichia pastoris
<400> 3
   tcatagtgca tcctcttgca ctattgacaa taatcgacga gttccagaga cgaggtcgca      60
   acaacgattc cataatattc ggtgggttac ttggtaaaca tgatgaatcc accaaccaaa     120
   tatctgttgt taacagcttt gtgataccat tgatcgataa tcagtttttg aataaagagt     180
   acttgcagga catgctactc aaattttcta tcattaattc caactttcga ttcgtaggtt     240
   actatcacgt tcaatcttta aacggtaccg aaactcaaca gtatgacttg aacgctatta     300
   acctagtatg ccaagatgat aataggcctt cgtcctttgt ccattggata gtaacagatc     360
   caaaagagtt caaatcattc tcgatgtatt acttggatga ttcaatggtt caactcgtca     420
   attccaatat tcaacattac atttctaaac cattgcccta tgaatttaaa aaccttctgt     480
   ctgagaaaat tgctatcgac acaatcctca agcaatccag gctagaaaaa gacttatcca     540
   ccaaaaactc actgaagaaa ttaaacaata gttatatcga cattcattcc tcactgaacg     600
   ttctctataa atcagtcaat aggcttattc gttacctcaa aaaatgctca aaatcagaag     660
   tttcaattga ctatgacaca gttcaggaaa tgaatactgt aatactgaaa attgaaaggc     720
   ttaaattgat accccaagtc aaggaggagt ttgacttagt gactctttca ctactggtag     780
   acaatcttga tcagatggat catcttttgt atctccggaa acaagtggaa cagtacaaaa     840
   tatctgaatc aatgtatagt tagtcaatat caaagtttca tacaacagtg accatccagc     900
   atcgcatctg tcagtttcgt ataatgtgcg cgcatgtgct gatcggtata tcaacatctc     960
   ttcctcttct ctgcttcctt tttcctcttg tttgttatca atgcagtacg taggtagagc    1020
   cattggatca gtatccaaaa cctggtcgtc cattaacccg gccacactga gtggtgctat    1080
   tgatatcatc gtggtggaac aagaaaatgg tgatctggct tgctctccct tccatgttcg    1140
   gtttggaaag tttcaattat tgaggccttc ccaaaagaaa gtggatttca tcgtcaatgg    1200
   agaaaagaca gacttaccaa tgaaactggg cgatgggggc gaagctttct ttgtgtttga    1260
   aaccgatgct gccatcccga gtgagcttca aacttccccc gtcatctcac ctgtatccag    1320
   cccagaacca gcctctccac tgagtactcc ttccagacca aactctgaac cagattattt 1380
   agaattaggg gatggagaat ctacaaccag tgaattagag aacttcaagt taaatcgata 1440
   tccctattta tcaactgagg tatcgcattc agatcctggt gttgggtctg tgagctcaag 1500
   tcctgagaat acgaagatta ttcaaaagat ctctcggaaa ctcaatacga aaaatattcc 1560
   ttccaaggtt gataataacg gaaatcttgt attggatata caaggttaca aaagtgatga 1620
   tctagacgac aattccaaat ctttgaaaca gttgttattg gctgaactcg gagaagatgt 1680
   agatttggac aaggttattg aaaaagatca cgaaggaaac attatgatca atggtgccat 1740
   atctttgttg agcggagaag atgatctgga atcatttcct cagaccgacg accaagctga 1800
   gagtctcaaa ttggatctgg agtcagataa atctgacatt gaatcagaca caaatcatga 1860
   attgtctcgc tacttcaaaa cgctaagact tacatcagat caattgaaat gtcttacact 1920
   gaagaaggga attaacgagc tgaaattcag tgtcaacaag ggcaaatctg tggtgacggc 1980
   aaatctatac ttctgggact attatgaccc catagtcata tcggatatag atggaaccat 2040
   tactaaatct gatgccttag gccatgtatt taccatgatt ggccgtgatt ggactcacaa 2100
   aggtgtagcc aaattatttt cagatatcaa atccaatgga tacaatatca tgtatttgac 2160
   tgcaaggtct gttggtcaag ccgattcgac ccgctactat ttaaacaaca ttgaacaaga 2220
   aggcttgagg cttcctcaag ggccggtgat cctttctccg gatcgcacaa tggccgcttt 2280
   acgaagagag gtaattttga agaaacctga ggtgttcaag atggcatgtt taaacgacat 2340
   aaagaaactg tatctcacca ataccaaaga cctaaatccg aacacagatt ctgcagattt 2400
   cacggatatc aatacgaaca ctttgagatc ttcaagtctt acagaggatg tacaaacgcc 2460
   attttacgcc ggttttggta atagaattac cgatgctttg tcctataggt cagtagggat 2520
   accatcatca agaattttta ccataaaccc agacggtgat gttcacatgg agctactcga 2580
   attggctggg tatagaagtt cttatgtaca tattagtgag ctggtagatc atttctttcc 2640
   accagtgaac actgaactat ttaagtcgat gccttctgat acctatcgga acacggcaaa 2700
   gttctctgat gtcaattatt ggaaagagcc tttgtacaat tttgaggagc tcagtgatga 2760
   agattctagt gaagatgagc taagaagaag aaaggaggag gaacgtttac agtctgctcc 2820
   tcgtagtcca atattggcag caggagcatc gtttttcaaa ggaagtagta gccttttagg 2880
   ttcaccagaa cggatgactt tgagtgaccc aaagccaact gaagttgccc ctagcacaat 2940
   caaaccccca aaatcagttg gttctgtatc atccgatgaa gaaaagctga aagatcatga 3000
   tgatttcatt gacgttgatc acgaagatga aaccttggat gatgatgacg atccctttga 3060
   ctacgattac gagtatgaag atgaagaaga ggaaaatgac gacgtagatg aagttgatga 3120
   tggggaagag tattcagatg attattatga cgaagaagac gactacgatg aagaattaga 3180
   tcatacttta gaaccagacc aaaagaaaga actggaccaa acagctgaag ccaaccagtt 3240
   acccccttca ggacccgatg aaatggaatc aaaatctttc aagaaagcca gtgatttaat 3300
   aagcaaaatg agaatcgatg acagctgagg gtaaataagt attagttaaa tctgcatttt 3360
   ctatttatat acattatttc tagagttctt tggttacttc aactggaatg gtggcgtcct 3420
   tttctctgtg tgactcggtt ttggcaaaga tttttttaag aacagcgtct tcgtcgtctt 3480
   cgtttctcat aataaatatc ttctttccat agctaaacca gtagaccaga gaaacaacca 3540
   accaaccaac aatagctgca gccgcgtagt tcatgttgga cttagtaact ggcttcacaa 3600
   atgggaatga gtagaatacc agagcaaata atgcccatgc aacggtcata ctggaggtga 3660
   aataaccaaa ctttccaatc cagaatggac catgagtgat actgtctctt ccattgtata 3720
   taagacaaga gacaggaatt gcataagaca acaggaggaa agtgatgcat ccggttacca 3780
   aagagttata agccaagtct gaagccaagt aaaggcaact gaccaaggaa cataaacaga 3840
   cggaaaacaa atgtgcgttg aatggaatac ccattctctt gtcgaccttt gaaaggaatg 3900
   gagaaaatgg aagaccattg tctctggcaa aagaccaaca gagtcgcatt tgccaggtgt 3960
   gaccagaaat taaacatcca caggcagtca aaaacaacaa gaaacccagg aaaatggcac 4020
   ctccttgatt ttgaagagct tggtagtaaa tggaaataat gggggcaccg gtagcagtgt 4080
   tcagcaatgc atccagatct ctgatagaga agaacatcga aatacagtac acaaaggaag 4140
   taaaaaatcc aatggcaaca gttcccatga tggcaattgg aataaccctt tcaggttggg 4200
   cgacttcttc agccatatgg gtagcacaat ccagacacga gaaggaccaa tttgggttaa 4260
   ttaaaccaat gatgaaggcg attccagcgg attcccaacc agtttggttg tcaaaatcgg 4320
   aaaac 4325
<210> 4
   <211> 775
   <212> PRT
   <213> Pichia pastoris
<400> 4
   Met Gln Tyr Val Gly Arg Ala Ile Gly Ser Val Ser Lys Thr Trp Ser
   1               5                   10                  15
   Ser Ile Asn Pro Ala Thr Leu Ser Gly Ala Ile Asp Ile Ile Val Val
               20                  25                  30
   Glu Gln Glu Asn Gly Asp Leu Ala Cys Ser Pro Phe His Val Arg Phe
           35                  40                  45
   Gly Lys Phe Gln Leu Leu Arg Pro Ser Gln Lys Lys Val Asp Phe Ile
       50                  55                  60
   Val Asn Gly Glu Lys Thr Asp Leu Pro Met Lys Leu Gly Asp Gly Gly
   65                  70                  75                  80
   Glu Ala Phe Phe Val Phe Glu Thr Asp Ala Ala Ile Pro Ser Glu Leu
                   85                  90                  95
   Gln Thr Ser Pro Val Ile Ser Pro Val Ser Ser Pro Glu Pro Ala Ser
               100                 105                 110
   Pro Leu Ser Thr Pro Ser Arg Pro Asn Ser Glu Pro Asp Tyr Leu Glu
           115                 120                 125
   Leu Gly Asp Gly Glu Ser Thr Thr Ser Glu Leu Glu Asn Phe Lys Leu
       130                 135                 140
   Asn Arg Tyr Pro Tyr Leu Ser Thr Glu Val Ser His Ser Asp Pro Gly
   145                 150                 155                 160
   Val Gly Ser Val Ser Ser Ser Pro Glu Asn Thr Lys Ile Ile Gln Lys
                   165                 170                 175
   Ile Ser Arg Lys Leu Asn Thr Lys Asn Ile Pro Ser Lys Val Asp Asn
               180                 185                 190
   Asn Gly Asn Leu Val Leu Asp Ile Gln Gly Tyr Lys Ser Asp Asp Leu
           195                 200                 205
   Asp Asp Asn Ser Lys Ser Leu Lys Gln Leu Leu Leu Ala Glu Leu Gly
       210                 215                 220
   Glu Asp Val Asp Leu Asp Lys Val Ile Glu Lys Asp His Glu Gly Asn
   225                 230                 235                 240
   Ile Met Ile Asn Gly Ala Ile Ser Leu Leu Ser Gly Glu Asp Asp Leu
                   245                 250                 255
   Glu Ser Phe Pro Gln Thr Asp Asp Gln Ala Glu Ser Leu Lys Leu Asp
               260                 265                 270
   Leu Glu Ser Asp Lys Ser Asp Ile Glu Ser Asp Thr Asn His Glu Leu
           275                 280                 285
   Ser Arg Tyr Phe Lys Thr Leu Arg Leu Thr Ser Asp Gln Leu Lys Cys
       290                 295                 300
   Leu Thr Leu Lys Lys Gly Ile Asn Glu Leu Lys Phe Ser Val Asn Lys
   305                 310                 315                 320
   Gly Lys Ser Val Val Thr Ala Asn Leu Tyr Phe Trp Asp Tyr Tyr Asp
                   325                 330                 335
   Pro Ile Val Ile Ser Asp Ile Asp Gly Thr Ile Thr Lys Ser Asp Ala
               340                 345                 350
   Leu Gly His Val Phe Thr Met Ile Gly Arg Asp Trp Thr His Lys Gly
           355                 360                 365
   Val Ala Lys Leu Phe Ser Asp Ile Lys Ser Asn Gly Tyr Asn Ile Met
       370                 375                 380
   Tyr Leu Thr Ala Arg Ser Val Gly Gln Ala Asp Ser Thr Arg Tyr Tyr
   385                 390                 395                 400
   Leu Asn Asn Ile Glu Gln Glu Gly Leu Arg Leu Pro Gln Gly Pro Val
                   405                 410                 415
   Ile Leu Ser Pro Asp Arg Thr Met Ala Ala Leu Arg Arg Glu Val Ile
               420                 425                 430
   Leu Lys Lys Pro Glu Val Phe Lys Met Ala Cys Leu Asn Asp Ile Lys
           435                 440                 445
   Lys Leu Tyr Leu Thr Asn Thr Lys Asp Leu Asn Pro Asn Thr Asp Ser
       450                 455                 460
   Ala Asp Phe Thr Asp Ile Asn Thr Asn Thr Leu Arg Ser Ser Ser Leu
   465                 470                 475                 480
   Thr Glu Asp Val Gln Thr Pro Phe Tyr Ala Gly Phe Gly Asn Arg Ile
                   485                 490                 495
   Thr Asp Ala Leu Ser Tyr Arg Ser Val Gly Ile Pro Ser Ser Arg Ile
               500                 505                 510
   Phe Thr Ile Asn Pro Asp Gly Asp Val His Met Glu Leu Leu Glu Leu
           515                 520                 525
   Ala Gly Tyr Arg Ser Ser Tyr Val His Ile Ser Glu Leu Val Asp His
       530                 535                 540
   Phe Phe Pro Pro Val Asn Thr Glu Leu Phe Lys Ser Met Pro Ser Asp
   545                 550                 555                 560
   Thr Tyr Arg Asn Thr Ala Lys Phe Ser Asp Val Asn Tyr Trp Lys Glu
                   565                 570                 575
   Pro Leu Tyr Asn Phe Glu Glu Leu Ser Asp Glu Asp Ser Ser Glu Asp
               580                 585                 590
   Glu Leu Arg Arg Arg Lys Glu Glu Glu Arg Leu Gln Ser Ala Pro Arg
           595                 600                 605
   Ser Pro Ile Leu Ala Ala Gly Ala Ser Phe Phe Lys Gly Ser Ser Ser
   610 615 620
   Leu Leu Gly Ser Pro Glu Arg Met Thr Leu Ser Asp Pro Lys Pro Thr
   625 630 635 640
   Glu Val Ala Pro Ser Thr Ile Lys Pro Pro Lys Ser Val Gly Ser Val
   645 650 655
   Ser Ser Asp Glu Glu Lys Leu Lys Asp His Asp Asp Phe Ile Asp Val
   660 665 670
   Asp His Glu Asp Glu Thr Leu Asp Asp Asp Asp Asp Pro Phe Asp Tyr
   675 680 685
   Asp Tyr Glu Tyr Glu Asp Glu Glu Glu Glu Asn Asp Asp Val Asp Glu
   690 695 700
   Val Asp Asp Gly Glu Glu Tyr Ser Asp Asp Tyr Tyr Asp Glu Glu Asp
   705 710 715 720
   Asp Tyr Asp Glu Glu Leu Asp His Thr Leu Glu Pro Asp Gln Lys Lys
   725 730 735
   Glu Leu Asp Gln Thr Ala Glu Ala Asn Gln Leu Pro Pro Ser Gly Pro
   740 745 750
   Asp Glu Met Glu Ser Lys Ser Phe Lys Lys Ala Ser Asp Leu Ile Ser
   755 760 765
   Lys Met Arg Ile Asp Asp Ser
   770 775
<210> 5
   <211> 858
   <212> DNA
   <213> Yarrowia lipolytica
<400> 5
   atgtctgctg catctactgg agctagcgcc accaagagtg cgcctgttag cagaaccgcc 60
   cagggcactc agtctacacc tgcttctctc aagagcgaaa ccaagactac cgaaaccaat 120
   tctaacatca ccgacaccaa cggcgaagac ctgttggcgt tctggcataa tggccggttt 180
   ggcgccttct tcaagaaaca cgaaatcccc agaaagttcc tccacacctc catcggcttc 240
   atcaccctat ggctgtacac ccaaggcatc aacctgaacc aggtcactcc cgttctcgtg 300
   gtgatggcat tgttcatcgg cacctttgac ttcctgcgtc tcagaaacaa ggccctcaac 360
   aaggcctact gcaaagtcat gggagctctg atgcgagagt ccgagattaa cggcgtgaac 420
   ggaaccatct ggtacctgct tggcctcatt ctggtcttct cgttcgcctc taaggatgtt 480
   gctcttatgg ctgttcttct gctctcctgg gctgataccg cagcctcttc cgtcggccgg 540
   gcttatggat acctcactcc gaagtacaag aacaagtcgc tggctggttt tatgggcgcc 600
   ttcattgcct ctgagatcgc atgtctcttt ctatacggca tcatcatccc caagtacggc 660
   gctgaagtcg acacgcccgg ccagatcacc tggtccaagc agtcttctcg acttactctg 720
   ggagtcttga cagccatgtc tggtctcgtc ggagccgttt ccgagtccat tgatgtgttc 780
   ggaatcgacg acaacttcac cattcccgtc ctctcggcgc tcttccttac tggtctgctg 840
   gacatcttca agaagtaa 858
<210> 6
   <211> 285
   <212> PRT
   <213> Yarrowia lipolytica
<400> 6
   Met Ser Ala Ala Ser Thr Gly Ala Ser Ala Thr Lys Ser Ala Pro Val
   1               5                   10                  15
   Ser Arg Thr Ala Gln Gly Thr Gln Ser Thr Pro Ala Ser Leu Lys Ser
               20                  25                  30
   Glu Thr Lys Thr Thr Glu Thr Asn Ser Asn Ile Thr Asp Thr Asn Gly
           35                  40                  45
   Glu Asp Leu Leu Ala Phe Trp His Asn Gly Arg Phe Gly Ala Phe Phe
       50                  55                  60
   Lys Lys His Glu Ile Pro Arg Lys Phe Leu His Thr Ser Ile Gly Phe
   65                  70                  75                  80
   Ile Thr Leu Trp Leu Tyr Thr Gln Gly Ile Asn Leu Asn Gln Val Thr
                   85                  90                  95
   Pro Val Leu Val Val Met Ala Leu Phe Ile Gly Thr Phe Asp Phe Leu
               100                 105                 110
   Arg Leu Arg Asn Lys Ala Leu Asn Lys Ala Tyr Cys Lys Val Met Gly
           115                 120                 125
   Ala Leu Met Arg Glu Ser Glu Ile Asn Gly Val Asn Gly Thr Ile Trp
       130                 135                 140
   Tyr Leu Leu Gly Leu Ile Leu Val Phe Ser Phe Ala Ser Lys Asp Val
   145                 150                 155                 160
   Ala Leu Met Ala Val Leu Leu Leu Ser Trp Ala Asp Thr Ala Ala Ser
                   165                 170                 175
   Ser Val Gly Arg Ala Tyr Gly Tyr Leu Thr Pro Lys Tyr Lys Asn Lys
               180                 185                 190
   Ser Leu Ala Gly Phe Met Gly Ala Phe Ile Ala Ser Glu Ile Ala Cys
           195                 200                 205
   Leu Phe Leu Tyr Gly Ile Ile Ile Pro Lys Tyr Gly Ala Glu Val Asp
       210                 215                 220
   Thr Pro Gly Gln Ile Thr Trp Ser Lys Gln Ser Ser Arg Leu Thr Leu
   225                 230                 235                 240
   Gly Val Leu Thr Ala Met Ser Gly Leu Val Gly Ala Val Ser Glu Ser
                   245                 250                 255
   Ile Asp Val Phe Gly Ile Asp Asp Asn Phe Thr Ile Pro Val Leu Ser
               260                 265                 270
   Ala Leu Phe Leu Thr Gly Leu Leu Asp Ile Phe Lys Lys
           275                 280                 285
<210> 7
   <211> 900
   <212> DNA
   <213> Pichia pastoris
<400> 7
   atggcagcaa ccacagcacg tcctcagaga ataggtcgct cgagagtgtc gaaattagaa 60
   gaaagaatat caacgtcttc ctcctattca tctctctcgt cgcttgatgc tagtgatgat 120
   accgtgttat ccaccgagtc aacaagccca gtcttagata aggctaaaaa tacatccacc 180
   tcgtatttgt tgttacaaag ccacaagagg ttcaataagt tgattcataa gcatgagtta 240
   cccaggaaac tattccatat gtccatcggg ttcttcacat tatacctgta cacaaagaat 300
   gtgcagaaag agcaaatcca gttacccctg gttgtgggat ttgtagtggt gtttgcactt 360
   gatttaataa ggttcagatg gcctcaagtt aacaaacttt attgttccac tgtggggttc 420
   ctcatgaggg agagagaggt ggacgagtat tataacggtg ttaactggta cctgctcggg 480
   ttggctgtag ttttcttgtt tttcaagaag gatattgcag tcatggcagt gctgttacta 540
   agtttatccg ataccgctgc ctccacggtc gggagagctt ggggccagta tacacccaag 600
   atcaccagcc acaaatcttt agctggttcg ttggccgctt gtgtcgttgg aatattgtca 660
   tgctacctct tttatggata tttcgttccg aactatcccg aatgcaatga cattccaaac 720
   cagttcgaat gggtttccga aaaaagtggc ctcaacattc attcattggc cctcctttct 780
   ggattggtcg ctagtgtaag tgaaggaatt gatctcttca actgggatga caacttcacc 840
   attccagttc tatcagctgt gtttttacga ctggttatca aactgacaaa tacagactaa 900
<210> 8
   <211> 299
   <212> PRT
   <213> Pichia pastoris
<400> 8
   Met Ala Ala Thr Thr Ala Arg Pro Gln Arg Ile Gly Arg Ser Arg Val
   1               5                   10                  15
   Ser Lys Leu Glu Glu Arg Ile Ser Thr Ser Ser Ser Tyr Ser Ser Leu
               20                  25                  30
   Ser Ser Leu Asp Ala Ser Asp Asp Thr Val Leu Ser Thr Glu Ser Thr
           35                  40                  45
   Ser Pro Val Leu Asp Lys Ala Lys Asn Thr Ser Thr Ser Tyr Leu Leu
       50                  55                  60
   Leu Gln Ser His Lys Arg Phe Asn Lys Leu Ile His Lys His Glu Leu
   65                  70                  75                  80
   Pro Arg Lys Leu Phe His Met Ser Ile Gly Phe Phe Thr Leu Tyr Leu
                   85                  90                  95
   Tyr Thr Lys Asn Val Gln Lys Glu Gln Ile Gln Leu Pro Leu Val Val
               100                 105                 110
   Gly Phe Val Val Val Phe Ala Leu Asp Leu Ile Arg Phe Arg Trp Pro
           115                 120                 125
   Gln Val Asn Lys Leu Tyr Cys Ser Thr Val Gly Phe Leu Met Arg Glu
       130                 135                 140
   Arg Glu Val Asp Glu Tyr Tyr Asn Gly Val Asn Trp Tyr Leu Leu Gly
   145                 150                 155                 160
   Leu Ala Val Val Phe Leu Phe Phe Lys Lys Asp Ile Ala Val Met Ala
                   165                 170                 175
   Val Leu Leu Leu Ser Leu Ser Asp Thr Ala Ala Ser Thr Val Gly Arg
               180                 185                 190
   Ala Trp Gly Gln Tyr Thr Pro Lys Ile Thr Ser His Lys Ser Leu Ala
           195                 200                 205
   Gly Ser Leu Ala Ala Cys Val Val Gly Ile Leu Ser Cys Tyr Leu Phe
       210                 215                 220
   Tyr Gly Tyr Phe Val Pro Asn Tyr Pro Glu Cys Asn Asp Ile Pro Asn
   225 230 235 240
   Gln Phe Glu Trp Val Ser Glu Lys Ser Gly Leu Asn Ile His Ser Leu
   245 250 255
   Ala Leu Leu Ser Gly Leu Val Ala Ser Val Ser Glu Gly Ile Asp Leu
   260 265 270
   Phe Asn Trp Asp Asp Asn Phe Thr Ile Pro Val Leu Ser Ala Val Phe
   275 280 285
   Leu Arg Leu Val Ile Lys Leu Thr Asn Thr Asp
   290 295
<210> 9
   <211> 600
   <212> DNA
   <213> Saccharomyces cerevisiae
<400> 9
   atggggaccg aagatgccat tgcccttcca aatagcacgc tagagccgcg taccgaagct 60
   aagcaaagac tatcatctaa gagtcatcaa gtctcggcga aagtaacgat tccagcaaaa 120
   gaagaaatta gtagtagcga tgacgatgca cacgttccag tgacagaaat acatttgaaa 180
   tctcatgaat ggttcggcga ttttataact aaacatgaaa ttccccgtaa ggtgttccat 240
   tcttccattg gctttattac tttgtacctg tatacgcagg gtattaatta taaaaatgtt 300
   ttatggcctt tgatatacgc cttcatcata ttgtttattt tggatctgat aagactaaac 360
   tggccctttt tcaatatgct ttactgtaga actgtgggtg cgctaatgag aaaaaaggag 420
   attcatacat acaatggggt attgtggtac attcttgggt taatcttttc ttttaacttt 480
   ttctctaaag atgttacctt aatatcgtta tttttgctaa gttggtccga tacagccgcc 540
   gcaactattg gaagaaagta tggtcattta acacccaaag tggcaagaaa taaatccctt 600
<210> 10
   <211> 290
   <212> PRT
   <213> Saccharomyces cerevisiae
<400> 10
   Met Gly Thr Glu Asp Ala Ile Ala Leu Pro Asn Ser Thr Leu Glu Pro
   1               5                   10                  15
   Arg Thr Glu Ala Lys Gln Arg Leu Ser Ser Lys Ser His Gln Val Ser
               20                  25                  30
   Ala Lys Val Thr Ile Pro Ala Lys Glu Glu Ile Ser Ser Ser Asp Asp
           35                  40                  45
   Asp Ala His Val Pro Val Thr Glu Ile His Leu Lys Ser His Glu Trp
       50                  55                  60
   Phe Gly Asp Phe Ile Thr Lys His Glu Ile Pro Arg Lys Val Phe His
   65                  70                  75                  80
   Ser Ser Ile Gly Phe Ile Thr Leu Tyr Leu Tyr Thr Gln Gly Ile Asn
                   85                  90                  95
   Tyr Lys Asn Val Leu Trp Pro Leu Ile Tyr Ala Phe Ile Ile Leu Phe
               100                 105                 110
   Ile Leu Asp Leu Ile Arg Leu Asn Trp Pro Phe Phe Asn Met Leu Tyr
           115                 120                 125
   Cys Arg Thr Val Gly Ala Leu Met Arg Lys Lys Glu Ile His Thr Tyr
       130                 135                 140
   Asn Gly Val Leu Trp Tyr Ile Leu Gly Leu Ile Phe Ser Phe Asn Phe
   145                 150                 155                 160
   Phe Ser Lys Asp Val Thr Leu Ile Ser Leu Phe Leu Leu Ser Trp Ser
                   165                 170                 175
   Asp Thr Ala Ala Ala Thr Ile Gly Arg Lys Tyr Gly His Leu Thr Pro
               180                 185                 190
   Lys Val Ala Arg Asn Lys Ser Leu Ala Gly Ser Ile Ala Ala Phe Thr
           195                 200                 205
   Val Gly Val Ile Thr Cys Trp Val Phe Tyr Gly Tyr Phe Val Pro Ala
       210                 215                 220
   Tyr Ser Tyr Val Asn Lys Pro Gly Glu Ile Gln Trp Ser Pro Glu Thr
   225                 230                 235                 240
   Ser Arg Leu Ser Leu Asn Met Leu Ser Leu Leu Gly Gly Val Val Ala
                   245                 250                 255
   Ala Leu Ser Glu Gly Ile Asp Leu Phe Asn Trp Asp Asp Asn Phe Thr
               260                 265                 270
   Ile Pro Val Leu Ser Ser Leu Phe Met Asn Ala Val Ile Lys Thr Phe
           275                 280                 285
   Lys Lys
       290
<210> 11
   <211> 5
   <212> PRT
   <213> Artificial Sequence
<220>
   <223> catalytic sequence
<400> 11
   Asp Ile Asp Gly Thr
   1               5
<210> 12
   <211> 30
   <212> DNA
   <213> Artificial Sequence
<220>
   <223> Primer
<400> 12
   gcggccgcga agacggtgag tatggccatc 30
<210> 13
   <211> 30
   <212> DNA
   <213> Artificial Sequence
<220>
   <223> Primer
<400> 13
   gcggccgccc aaaccatgca tacaaatcag 30
<210> 14
   <211> 44
   <212> DNA
   <213> Artificial Sequence
<220>
   <223> Primer
<400> 14
   aaaaacaact aattattcga aatggcagca accacagcac gtcc 44
<210> 15
   <211> 45
   <212> DNA
   <213> Artificial Sequence
<220>
   <223> Primer
<400> 15
   aaggcgaatt aattcgcggc cgcttagtct gtatttgtca gtttg 45
<210> 16
   <211> 26
   <212> DNA
   <213> Artificial Sequence
<220>
   <223> Primer
<400> 16
   ttcgaaatgg gtgcgagagc gtcagt 26
<210> 17
   <211> 26
   <212> DNA
   <213> Artificial Sequence
<220>
   <223> Primer
<400> 17
   gcggccgctt attgtgacga ggggtc 26
<210> 18
   <211> 40
   <212> DNA
   <213> Artificial Sequence
<220>
   <223> Primer
<400> 18
   tagggataac agggtaatcc attgacacag aactcgacct 40
<210> 19
   <211> 40
   <212> DNA
   <213> Artificial Sequence
<220>
   <223> Primer
<400> 19
   attaccctgt tatccctacc gtactgtaca ccgtagtttg 40
<210> 20
   <211> 34
   <212> DNA
   <213> Artificial Sequence
<220>
   <223> Primer
<400> 20
   agatcttaat aagccacact gagtggtgct attg 34
<210> 21
   <211> 35
   <212> DNA
   <213> Artificial Sequence
<220>
   <223> Primer
<400> 21
   gcggccgctt acgtattctc aggacttgag ctcac 35